# FASCICULE DE BREVET EUROPEEN

(11) **EP 3 768 828 B1**
(45) Date de publication et mention de la délivrance du brevet: **09.12.2026**
(21) Numéro de dépôt: 19718442.7
(22) Date de dépôt: 22.03.2019
(51) Int. Cl.: A61K 40/41, A61K 40/22, A61K 40/11, C12N 5/0783, A61K 40/32

(54) **NOUVELLE MÉTHODE D'OBTENTION DE CELLULES T À PARTIR DE CELLULES SOUCHES PLURIPOTENTES, ET LEURS UTILISATIONS**
NEUARTIGES VERFAHREN ZUR HERSTELLUNG VON T-ZELLEN AUS PLURIPOTENTEN STAMMZELLEN UND VERWENDUNGEN DAVON
NOVEL METHOD FOR OBTAINING T CELLS FROM PLURIPOTENT STEM CELLS, AND USES THEREOF

(30) Priorité: 23.03.2018 FR 1852531
(43) Date de publication de la demande: 27.01.2021
(73) Titulaire: Centre National de la Recherche Scientifique, 75016 Paris (FR); INSERM (Institut National de la Santé et de la Recherche Médicale), 75013 Paris (FR); Centre Hospitalier Universitaire De Nantes, 44000 Nantes (FR); Nantes Université, 44300 Nantes (FR)
(72) Inventeur: GUILLONNEAU, Carole, 44118 La Chevrolière (FR); DAVID, Laurent, 44100 Nantes (FR); FLIPPE, Léa, 44420 Piriac sur Mer (FR)
(74) Mandataire: Icosa
(86) Numéro de dépôt international: PCT/FR2019/050667
(87) Numéro de publication internationale: WO 2019/180394

(56) Documents cités:
- WO-A1-2010/051634
- WO-A1-2017/100403
- WO-A2-2014/165707
- CHIA-WEI CHANG ET AL: "Broad T-Cell Receptor Repertoire in T-Lymphocytes Derived from Human Induced Pluripotent Stem Cells", PLOS ONE, vol. 9, no. 5, 14 May 2014 (2014-05-14), pages e97335, XP055539276, DOI: 10.1371/journal.pone.0097335
- LEI F ET AL: "T lineage differentiation from induced pluripotent stem cells", CELLULAR IMMUNOLOGY, ACADEMIC PRESS, SAN DIEGO, CA, US, vol. 260, no. 1, 1 January 2009 (2009-01-01), pages 1 - 5, XP026733960, ISSN: 0008-8749, [retrieved on 20090915], DOI: 10.1016/J.CELLIMM.2009.09.005
- R. HOLMES ET AL: "The OP9-DL1 System: Generation of T-Lymphocytes from Embryonic or Hematopoietic Stem Cells In Vitro", COLD SPRING HARBOR PROTOCOLS, vol. 2009, no. 2, 1 February 2009 (2009-02-01), XP055332733, DOI: 10.1101/pdb.prot5156
- R. HAQUE ET AL: "Programming of Regulatory T Cells from Pluripotent Stem Cells and Prevention of Autoimmunity", THE JOURNAL OF IMMUNOLOGY, vol. 189, no. 3, 25 June 2012 (2012-06-25), US, pages 1228 - 1236, XP055377907, ISSN: 0022-1767, DOI: 10.4049/jimmunol.1200633
- MOHAMMAD HAQUE ET AL: "Stem cell-derived tissue-associated regulatory T cells ameliorate the development of autoimmunity", SCIENTIFIC REPORTS, vol. 6, no. 1, 5 February 2016 (2016-02-05), XP055596139, DOI: 10.1038/srep20588

## Description

### DOMAINE DE L'INVENTION

L'invention appartient au domaine technique de l'immunologie. Plus particulièrement, l'invention concerne une méthode pour obtenir des cellules T comprenant des cellules T régulatrices (Treg), à partir de cellules souches pluripotentes.

### ÉTAT DE LA TECHNIQUE

Les lymphocytes T, ou cellules T, sont des cellules qui jouent un rôle central dans la réponse immunitaire secondaire. Ils tiennent leur nom de leur différenciation qui se produit dans le thymus. A leur entrée dans cet organe, ils perdent rapidement leur potentiel à se différencier en lymphocyte B, en raison de l'interaction des progéniteurs exprimant Notch1, avec les ligands Delta-1 et Delta-4 de Notch, fortement et exclusivement exprimés dans le thymus (Cavazzana-Calvo 2007). Parmi les cellules T, différentes sous-populations ont été identifiées, notamment les lymphocytes T régulateurs « Treg », les lymphocytes T effecteur « Teff » et les lymphocytes T *Natural Killer* « NKT ».

Les lymphocytes T peuvent par exemple être identifiés par l'expression de CD3, CD4, CD8 et/ou du récepteur à cellules T « TCR ».

Les cellules T régulatrices, dites cellules Treg, sont habituellement caractérisées par l'expression de Foxp3 (*Forkhead box P3*), un facteur de transcription essentiel à leur différenciation lors de l'hématopoïèse. Les cellules Treg représentent 5 à 10 % de la sous-population de cellules T CD4+ chez la souris, le rat et l'humain, avec environ 1 à 2 % de ces cellules Treg circulant dans la circulation périphérique (Haque 2016). La grande capacité des cellules Treg à contrôler la réponse immunitaire a conduit à les considérer comme des outils de contrôle de la réponse immunitaire non-souhaitée, notamment dans le contexte des transplantations, greffes ou pour traiter des maladies auto-immunes et inflammatoires. Des essais cliniques ont ainsi déjà été conduits et ont montré des résultats encourageants pour des thérapies cellulaires avec les Treg, par exemple dans le traitement de la GvHD (réaction du greffon contre l'hôte), suite à une transplantation de cellules souches hématopoïétiques allogéniques, la transplantation d'organes, chez les patients souffrant de diabète de type 1, les maladies inflammatoires chroniques, ainsi que chez des patients ayant subi une greffe de rein ou de foie (Passerini 2017).

Les lymphocytes T effecteurs sont capables de produire différentes cytokines et d'adopter une activité cytotoxique leur permettant de reconnaître et détruire les cellules considérées comme étrangères à l'organisme. Elles jouent un rôle important dans le rejet des greffes et dans la mort de cellules tumorales ou de cellules infectées par des virus.

Les lymphocytes NKT constituent 0,2 % environ des lymphocytes T du sang périphérique. Ils ont la particularité de présenter à la fois des caractères propres aux cellules T, tels que l'expression d'un TCR ou la réponse de type spécifique aux antigènes, et des caractères associés aux cellules *Natural Killer «* NK » de l'immunité innée, tels que l'expression de NK1.1 ou CD56 (Kato 2018).

L'utilisation thérapeutique des cellules T est en plein essor. Il existe donc un besoin pour des méthodes de culture permettant l'obtention de cellules T efficaces et en quantité suffisante pour permettre leur application thérapeutique.

Plusieurs protocoles permettant de générer des cellules T à partir de cellules pluripotentes ont été mis en œuvre et sont notamment décrits par les demandes internationales WO 2017/100403, WO 2014/165707, WO 2010/051634, ainsi que par les publications scientifiques de Chang et al. (2014), Lei et al. (2009) and Holmes et al. (2009).

Il est connu que la mise en culture des cellules souches pluripotentes en trois dimensions, par exemple dans un biomatériau comme une matrice d'hydrogel, permet de reproduire de façon proche les interactions et contraintes physiques qui s'exercent lors du développement normal de l'embryon. Ces conditions « améliorées » aboutissent en l'obtention de populations cellulaires aux phénotypes plus représentatifs de ceux observés lors du développement et de la différenciation dans un organisme vivant.

Par ailleurs, l'introduction du gène FOXP3 associé à celui permettant l'expression du TCR, par le biais de lentivirus, dans des cellules souches pluripotentes induites murines mises en co-culture avec des cellules exprimant les ligands de Notch (lignées OP9-DLL1 et OP9-DLL4) ont permis la production de cellules Treg CD4+CD3+CD25+ Foxp3+ ou CD8+CD3+CD25+Foxp3+, et portant le TCR (TCR+) (Haque 2012 ; Haque 2016). Ces résultats obtenus chez la souris ont impliqué l'utilisation de fragments de thymus, ce qui ne serait pas envisageable pour une application thérapeutique chez l'humain.

Ainsi il existe un besoin pour un protocole permettant de générer, à partir de cellules souches pluripotentes, des cellules T, notamment des cellules Treg, directement utilisables en thérapie chez l'humain et ne mettant pas en œuvre des fragments de thymus.

Par ailleurs, il n'existe pas, à la connaissance actuelle des inventeurs, de méthodes publiées permettant d'obtenir une expression significative du TCR dans les populations de cellules Teff et de cellules T CD4-CD8-CD3+TCRab+.

De plus, il existe un besoin pour un protocole permettant d'obtenir une population de cellules T comprenant à la fois des cellules Treg, Teff et des cellules T CD4-CD8-CD3+TCRab+.

Ainsi, il existe un besoin pour une méthode simple permettant d'obtenir des cellules T, notamment des Treg, Teff et des cellules T CD4-CD8-CD3+TCRab+, en grand nombre, notamment pour des applications thérapeutiques. Il existe notamment un besoin pour une méthode simple permettant d'obtenir des cellules T en grand nombre exprimant un récepteur antigène chimérique ou « CAR » (*Chimeric Antigen Receptor*) pouvant être utilisées dans une thérapie cellulaire, par exemple pour le traitement d'un rejet de greffe, de la réaction du greffon contre l'hôte, d'une maladie auto-immune, d'une maladie inflammatoire ou d'un cancer. En particulier, il existe un besoin pour une méthode simple permettant d'obtenir des cellules Treg en grand nombre exprimant un récepteur antigène chimérique ou « CAR » (*Chimeric Antigen Receptor*)*,* par exemple un CAR anti-HLA, pouvant être utilisées dans une thérapie cellulaire, par exemple pour le traitement d'un rejet de greffe ou de la réaction du greffon contre l'hôte.

### RÉSUMÉ

La présente invention concerne une méthode pour obtenir une population de cellules T comprenant des cellules T régulatrices à partir de cellules souches pluripotentes, comprenant les étapes suivantes :
a) cultiver des cellules souches pluripotentes dans une milieu de culture exempt de sérum, comprenant du BMP, du FGF2, du VEGF, du SCF, du Flt3-L et/ou de l'IL-3, dans des conditions permettant d'obtenir des corps embryoïdes comprenant au moins 5 % de cellules CD34+CD43+ ;
b) dissocier lesdits corps embryoïdes ;
c) placer lesdits corps embryoïdes dissociés en culture, dans un milieu de culture comprenant du SCF, du Flt3-L et/ou de l'IL-7, avec au moins un ligand de Notch, de préférence en coculture avec des cellules exprimant au moins un ligand de Notch ;
d) réaliser, au cours l'étape c), l'introduction entre le jour 8 (Jd8) et le jour 12 (Jd12) d'un vecteur comprenant au moins une séquence d'acide nucléique codant pour Foxp3 dans au moins une cellule, de préférence quand au moins 15 % de la population cellulaire présente le phénotype CD43- .

Selon un mode de réalisation, les corps embryoïdes obtenus à l'étape a) comprennent au moins 15% de cellules CD34+CD43+.

Selon un mode de réalisation, l'étape a) est réalisée pendant au moins 9 jours, de préférence pendant 9 à 12 jours.

Selon un mode de réalisation, les corps embryoïdes dissociés, obtenus à l'issue de l'étape b), sont placés en culture pendant au moins 15 jours.

Selon un mode de réalisation, le vecteur de l'étape d) est un rétrovirus, de préférence un lentivirus.

Selon un mode de réalisation, la séquence d'acide nucléique du vecteur de l'étape d) comprend une séquence d'acide nucléique d'intérêt supplémentaire.

Selon un mode de réalisation, l'acide nucléique d'intérêt supplémentaire est choisi parmi un acide nucléique codant pour un récepteur antigénique chimérique (CAR), pour Mcl-1, pour Bcl-xL, et pour Helios.

Selon un mode de réalisation, les cellules souches pluripotentes sont des cellules souches pluripotentes induites humaines ou des cellules souches embryonnaires humaines.

Selon un mode de réalisation, la méthode selon l'invention comprend en outre une étape d'isolement d'une population de cellules choisie parmi les cellules T régulatrices (Treg), les lymphocytes T effecteurs (Teff), et les lymphocytes T CD4-CD8-CD3+TCRab+.

Selon un mode de réalisation, la population de cellules T obtenue selon la méthode comprend des cellules T régulatrices (Treg), des cellules T effectrices (Teff) et des cellules T CD4-CD8-CD3+TCRab+.

Selon un mode de réalisation, la population de cellules T obtenue selon la méthode comprend des cellules Treg CD8+CD3+TCRab+Foxp3+ et CD4+CD3+TCRab+Foxp3+, des cellules T effectrices CD8+ et CD4+, et des cellules T CD4-CD8-CD3+TCRab+.

### DÉFINITIONS

Dans le contexte de l'invention, le terme « **environ** » est utilisé pour indiquer qu'une valeur comprend les variations inhérentes à la marge d'erreur liée à l'utilisation d'un appareil de mesure, à la méthode employée pour déterminer la valeur, ou les variations pouvant exister entre les cellules au sein de la population étudiée et entre les populations. Ainsi, dans un mode de réalisation, le terme « environ », placé devant une valeur, correspond à ± 10% de cette valeur.

Dans le contexte de l'invention, les termes « **corps embryoïdes** » quand ils se rapportent à des cellules, désignent des structures ou agrégats en trois dimensions qui comprennent une population de cellules souches pluripotentes qui ont subi une différenciation. Des méthodes de l'art connues pour permettre le développement de corps embryoïdes sont par exemple décrites dans WO2006021950 et Pettinato (Pettinato et al. 2015. Engineering Strategies for the Formation of Embryoid Bodies from Human Pluripotent Stem Cells. Stem Cells Dev. 24(14):1595-609).

Dans le contexte de l'invention, les termes « **vecteur** » se réfèrent à une macromolécule ou à un complexe de molécules comprenant un polynucléotide à délivrer dans une cellule hôte. Selon un mode de réalisation, le vecteur est choisi dans le groupe comprenant les vecteurs adénovirus, plasmides, vecteurs viraux adéno-associés, les vecteurs rétroviraux, les vecteurs viraux adéno-associés hybrides, les vecteurs lentivirus, les vecteurs viraux herpes simplex, les vecteurs vaccins, ou un liposome pégylé décoré, portant des anticorps spécifiques à la cellule hôte ciblée. Selon un mode de réalisation, le vecteur est un virus, particulièrement un rétrovirus, plus particulièrement un lentivirus. Le terme « **plasmide** » désigne un type commun de vecteur, qui est une molécule d'ADN extrachromosomique séparée de l'ADN chromosomique et qui est capable de se répliquer indépendamment de l'ADN chromosomique. Dans certains cas, il est circulaire et en double brin.

Dans le contexte de l'invention, les termes « **gène** », « **polynucléotide** », « **région codante** », « **séquence d'acide nucléique** », qui code pour une protéine particulière, est une molécule d'acide nucléique qui est transcrite et optionnellement aussi traduite en un produit de gène, *e.g.* un polypeptide. La région codante peut être présente soit sous la forme de ADNc, ADN génomique, ou ARN. Quand présente sous la forme d'un ADN, la molécule d'acide nucléique peut être simple brin (*i.e.* brin sens) ou double brin.

Dans le contexte de l'invention, le terme « **transduction** » est utilisé pour désigner un mode d'introduction d'un vecteur comprenant une séquence d'acide nucléique d'intérêt dans au moins une cellule d'une population cellulaire « **transduite** ». Plus particulièrement, le terme « transduction » désigne l'introduction d'un vecteur viral, de préférence rétroviral, de préférence lentiviral, comprenant une séquence d'acide nucléique d'intérêt dans au moins une cellule d'une population cellulaire.

Dans le contexte de l'invention, le terme « **Foxp3** » désigne le gène *Forkhead box P3* (symbole officiel FOXP3, ID : 50943 chez l'humain), ainsi que l'ARNm et la protéine codés par ce gène. La protéine exprimée à partir de ce gène est un facteur de transcription appartenant à la famille des protéines FOX. Elle est également appelée scurfine.

Dans le contexte de l'invention, l'expression « **phénotype** » désigne la présence ou l'absence d'un marqueur particulier, notamment à la surface de la cellule, ou d'un ensemble de cellules au sein de la population. Par exemple, le phénotype « CD34+CD43+ » désigne une cellule, ou un ensemble de cellules dans la population, qui exprime CD34 et CD43. « CD43- » désigne une cellule, ou un ensemble de cellules dans la population, qui n'exprime pas CD43. CD43, également connu sous les noms de Ly-48, leucosialine, sialophorine, leucocyte sialoglycoprotéine, et W3/13, est une glycoprotéine transmembranaire de type I comprenant de nombreux sites de O-glycosylation et sialylation. Le phénotype peut être mis en évidence par des moyens connus de l'art. Par exemple, un anticorps spécifique de CD43 conjugué à l'allophycocyanine (APC) peut être utilisé pour mesurer l'expression de CD43 dans une population de cellules par cytométrie en flux, et identifier le phénotype « CD43- » ou « CD43+ ». Dans un autre exemple, le phénotype « CD34+ » peut être mis en évidence par l'utilisation d'un anticorps spécifique au CD34 conjugué à au complexe phycoérythrine-cyanine7 (PE-cy7), qui se liera aux cellules porteuses du marqueur CD34, et qui pourra être quantifié par cytométrie en flux. CD34, également nommé « gp105-120 », est une phosphoglycoprotéine transmembranaire d'environ 105 à 120 kD, appartenant à la famille des sialomucines.

Dans le contexte de l'invention, la lettre « **J** » suivie d'un nombre désigne un jour particulier au cours de la première phase de culture cellulaire du procédé de l'invention (phase d'obtention de corps embryoïdes comprenant des cellules souches hématopoïétiques). Ainsi, J0 correspond au début de la première phase, J1 est le jour suivant J0 dans la première phase, etc. Les lettres « **Jd** » suivies d'un nombre désignent un jour particulier au cours de la seconde phase de culture cellulaire du procédé de l'invention (phase de différenciation en cellules T). Ainsi, Jd0 est le début de la seconde phase, Jd1 est le jour suivant Jd0 dans la seconde phase, etc.

Dans le contexte de l'invention, le terme « **traitement** » désigne la thérapie, la prévention, le retardement ou la réduction des symptômes provoqués par ou causés par une pathologie. En particulier, le terme « traitement » inclut le contrôle de la progression de la pathologie et des symptômes associés. L'efficacité du traitement d'une pathologie en lien avec un dérèglement immun correspond à une réduction de la réponse immunitaire non désirée, ou une augmentation de la réponse immunitaire déficiente. Plus généralement, le traitement d'une pathologie en lien avec un dérèglement immun permet de rétablir l'homéostasie immunitaire (*i.e.* réduire l'intensité de la réponse immunitaire lorsque celle-ci est trop forte, ou accroître l'amplitude de la réponse immunitaire lorsque celle-ci est trop faible ou nulle). Le terme « **traitement anti-rejet** » désigne un traitement pour éviter le rejet de greffe (traitement d'un rejet de greffe). Le terme « **traitement anti-cancer** » désigne un traitement pour le cancer.

### BRÈVE DESCRIPTION DES FIGURES

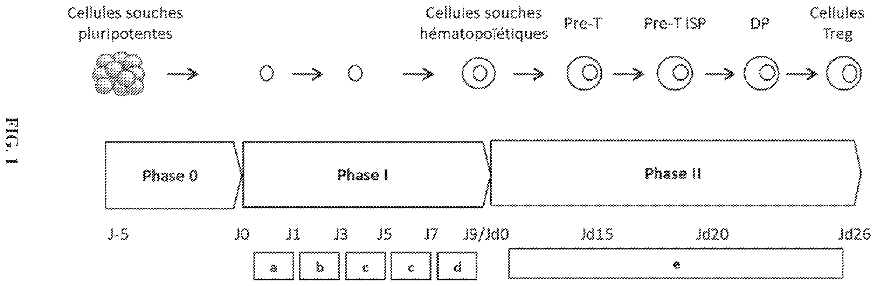

**Figure 1** **: représentation schématique du protocole expérimental** de différenciation cellulaire selon un mode de réalisation particulier. La composition des milieux de culture est précisée dans la partie expérimentale. **Pré-T** : préthymocytes ; **Pré-T ISP** : préthymocytes immatures simples positifs ; **DP** : thymocytes doubles positifs ; **Treg** : cellules T régulatrices. Les lettres encadrées **a, b, c, d** et **e** correspondent chacune à un protocole de renouvellement du milieu de culture et/ou de réensemencement des cellules, dont le détail est donné dans la partie expérimentale.

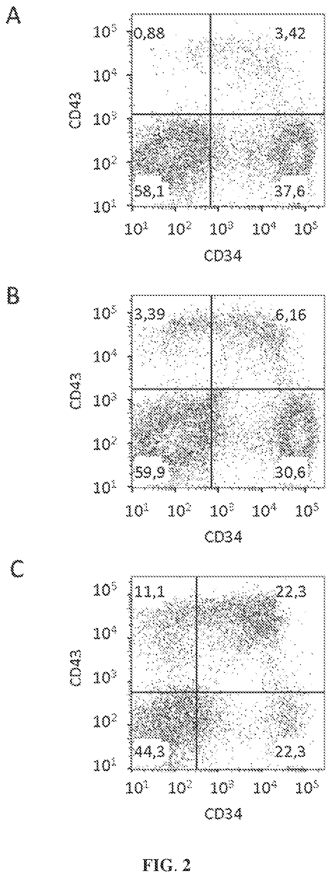

**Figure 2** **: phénotypes cellulaires des corps embryoïdes à J7, J8 et J9 ;** histogrammes biparamétriques pour la présence de CD34 et CD43, marqueurs des cellules souches hématopoïétiques. **A** : mesure à J7. **B** : mesure à J8. **C** : mesure à J9.

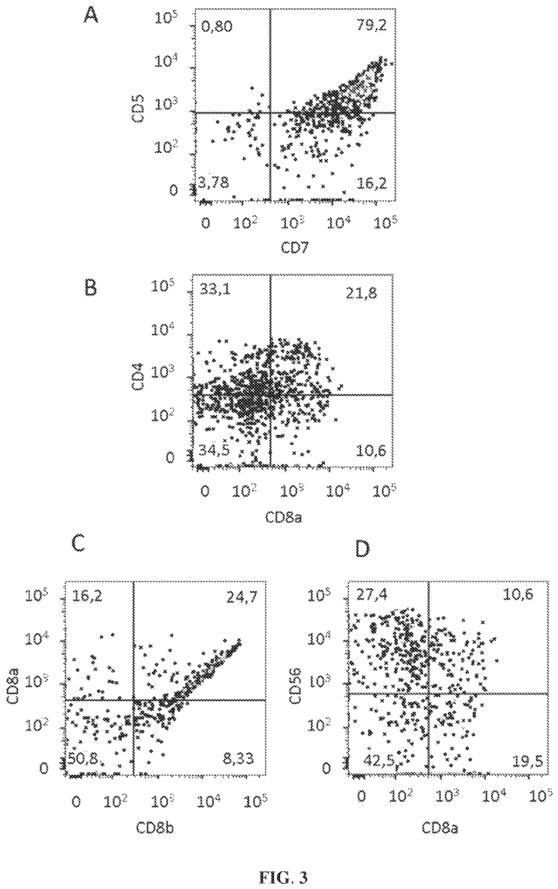

**Figure 3** **: phénotypes cellulaires au jour 20 de la phase de différenciation (Jd20)** ; histogrammes biparamétriques pour la présence à Jd20 de CD4, CD5, CD7, CD8a, CD8b et CD56 dans la population cellulaire pour laquelle la mise en coculture a été effectuée à J9. **A :** expression de CD5 et CD7, marqueurs des cellules lymphoïdes. **B** : expression de CD4 et CD8a au sein de la sous-population CD5+CD7+ identifiée en **A. C :** expression de CD8a et CD8b au sein de la sous-population CD5+CD7+ identifiée en **A. D :** expression de CD56 et CD8a au sein de la sous-population CD5+CD7+ identifiée en **A.**

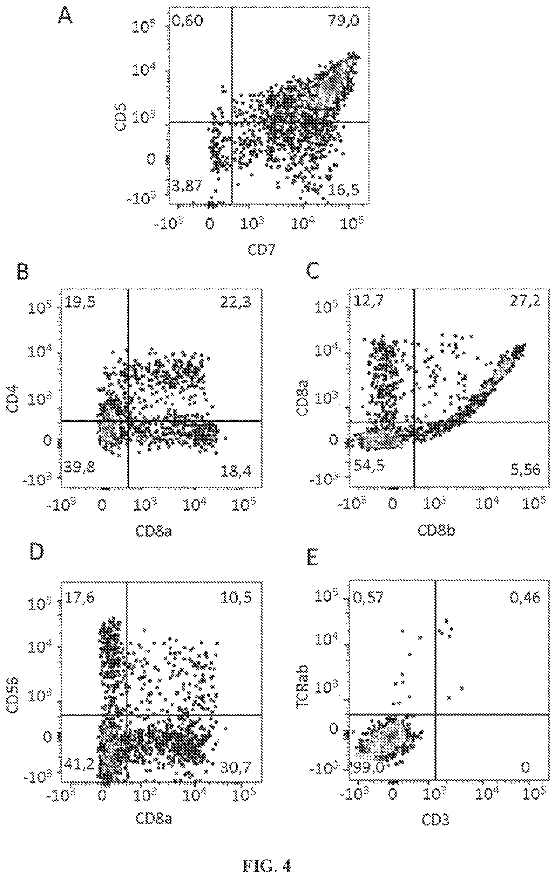

**Figure 4** **: phénotypes cellulaires au jour 26 de la phase de différenciation (Jd26)** : histogrammes biparamétriques pour la présence à Jd26 de CD3, CD4, CD5, CD7, CD8a, CD8b, CD56 et TCRab dans la population cellulaire pour laquelle la mise en coculture a été effectuée à J9. **A** : expression de CD5 et CD7, marqueurs des cellules lymphoïdes. **B** : expression des marqueurs des cellules T CD4 et CD8a au sein de la sous-population CD5+CD7+ identifiée en **A. C** : expression des marqueurs des cellules T CD8a et CD8b au sein de la sous-population CD5+CD7+ identifiée en **A. D** : expression des marqueurs des cellules NK et T CD56 et CD8a au sein de la sous-population CD5+CD7+ identifiée en **A. E** : expression des marqueurs des cellules T CD3 et TCRab au sein de la sous-population CD5+CD7+ identifiée en **A.**

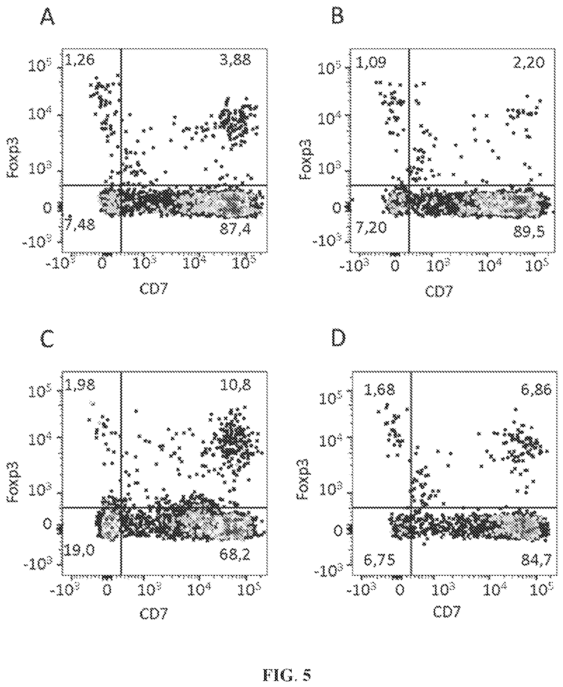

**Figure 5** **: phénotypes cellulaires au jour 26 de la phase de différenciation (Jd26)** : histogrammes biparamétriques pour la présence à Jd26 de Foxp3 et CD7 dans la population cellulaire pour laquelle la mise en coculture a été effectuée à J9 et la transduction de Foxp3 a eu lieu à différents temps à compter de la mise en coculture. **A** : résultats pour transduction à Jd0. **B** : résultats pour transduction à Jd5. **C** : résultats pour transduction à Jd10. **D :** résultats pour transduction à Jd15.

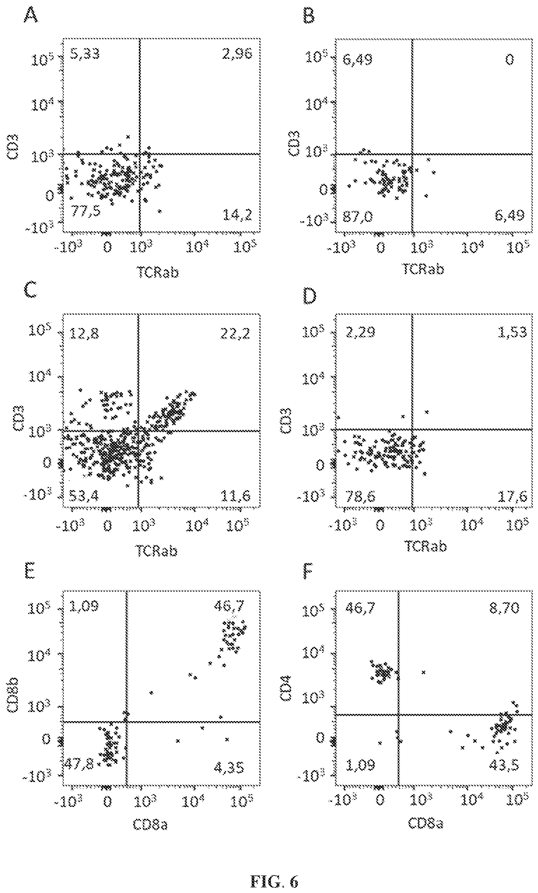

**Figure 6** **: phénotypes cellulaires au jour 26 de la phase de différenciation (Jd26)** : histogrammes biparamétriques pour la présence à Jd26 de CD3, CD4, CD8a, CD8b et TCRab dans les sous-populations cellulaires **Foxp3+CD7+** identifiées en figure 5. **A** : expression de CD3 et TCRab quand la transduction a eu lieu à Jd0. **B** : expression de CD3 et TCRab quand la transduction a eu lieu à Jd5. **C** : expression de CD3 et TCRab quand la transduction a eu lieu à Jd10. **D** : expression de CD3 et TCRab quand la transduction a eu lieu à Jd15. **E :** expression de CD8a et CD8b dans la sous-population CD3+TCRab+ identifiée en **C** (transduction à Jd10). **F** : expression de CD8a et CD4 dans la sous-population CD8a+CD8b+ identifiée en **E** (transduction à Jd10).

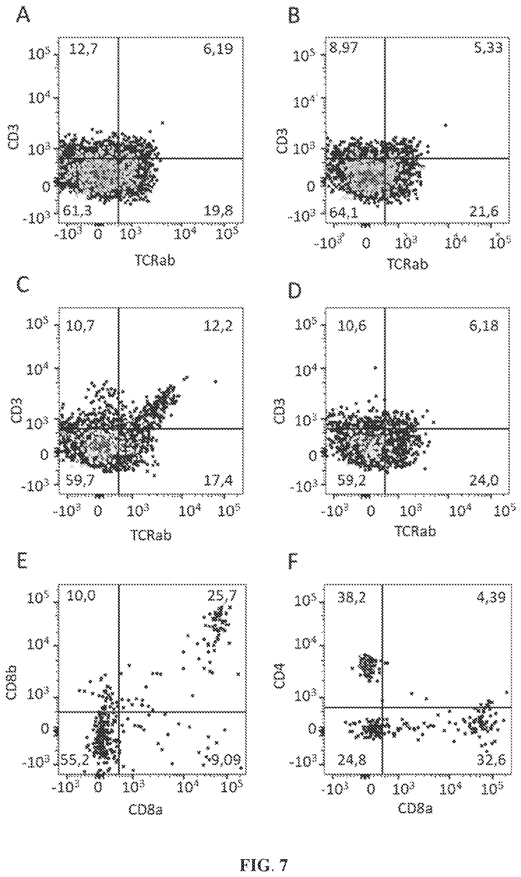

**Figure 7** **: phénotypes cellulaires au jour 26 de différenciation (Jd26)** : histogrammes biparamétriques pour la présence à Jd26 de CD3, CD4, CD8a, CD8b et TCRab dans les sous-populations cellulaires **Foxp3-CD7+** identifiées en figure 5. **A** : expression de CD3 et TCRab quand la transduction a eu lieu à Jd0. **B** : expression de CD3 et TCRab quand la transduction a eu lieu à Jd5. **C** : expression de CD3 et TCRab quand la transduction a eu lieu à Jd10. **D :** expression de CD3 et TCRab quand la transduction a eu lieu à Jd15. **E** : expression de CD8a et CD8b dans la sous-population CD3+TCRab+ identifiée en C (transduction à Jd10). **F :** expression de CD8a et CD4 dans la sous-population CD8a+CD8b+ identifiée en **E** (transduction à Jd10).

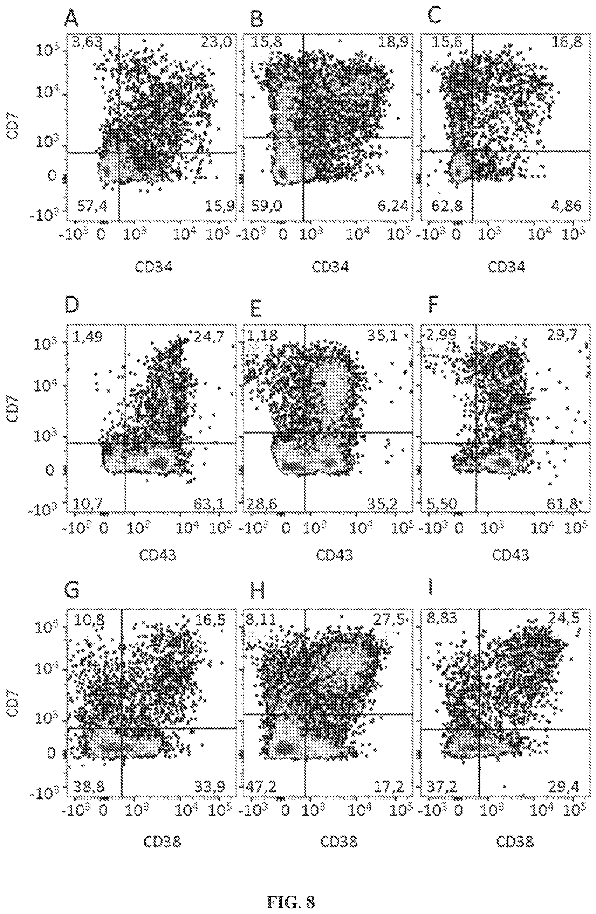

**Figure 8** **: phénotypes cellulaires au jour 26 de différenciation (Jd26)** : histogrammes biparamétriques pour la présence de CD7, CD34, CD38, CD43 dans la population cellulaire pour laquelle la mise en coculture a été effectuée à J9, en l'absence de transduction. **A** : expression de CD7 et CD34 à **Jd8. B :** expression de CD7 et CD34 à **Jd10. C** : expression de CD7 et CD34 à **Jd12. D** : expression de CD7 et CD43 à **Jd8. E :** expression de CD7 et CD43 à **Jd10. F :** expression de CD7 et CD43 à **Jd12. G** : expression de CD7 et CD38 à **Jd8. H** : expression de CD7 et CD38 à **Jd10. I :** expression de CD7 et CD38 à **Jd12.**

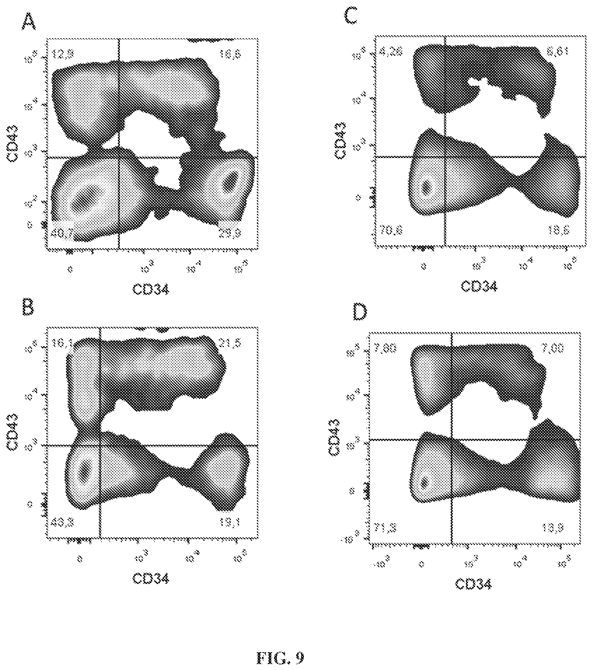

**Figure 9** **: phénotypes cellulaires des corps embryoïdes à J9, obtenus à partir de lignées cellulaires hES et de lignées cellulaires hiPS distinctes** : histogrammes biparamétriques pour la présence de CD34 et CD43, marqueurs des cellules souches hématopoïétiques. **A** : hES WA09. **B** : hiPS T04. **C** : hiPS LON80. **D** : hES WA01.

### DESCRIPTION DÉTAILLÉE

Les inventeurs ont mis en évidence une nouvelle méthode pour obtenir une population de lymphocytes T comprenant des cellules T régulatrices à partir de cellules souches pluripotentes. Plus particulièrement, la population de cellules T comprenant des cellules T régulatrices obtenue selon la méthode de l'invention peut comprendre des cellules Treg CD8+CD3+TCRab+Foxp3+ et/ou CD4+CD3+TCRab+Foxp3+, des cellules Teff (Foxp3-) CD8+ et/ou CD4+, et des cellules T CD4-CD8-CD3+TCRab+.

La présente invention se rapporte à une méthode pour obtenir une population de cellules T comprenant des cellules T régulatrices (Treg) à partir de cellules souches pluripotentes, comprenant les étapes suivantes :
a) cultiver des cellules souches pluripotentes dans un milieu de culture exempt de sérum, comprenant du BMP, du FGF2, du VEGF, du SCF, du Flt3-L et/ou de l'IL-3, dans des conditions permettant d'obtenir des corps embryoïdes comprenant au moins 5 % de cellules CD34+CD43+ ;
b) dissocier lesdits corps embryoïdes ;
c) placer lesdits corps embryoïdes dissociés en culture, dans un milieu de culture comprenant du SCF, du Flt3-L et/ou de l'IL-7, avec au moins un ligand de Notch, de préférence en coculture avec des cellules exprimant au moins un ligand de Notch ;
d) réaliser, au cours l'étape c), l'introduction entre le jour 8 (Jd8) et le jour 12 (Jd12) d'un vecteur comprenant au moins une séquence d'acide nucléique codant pour Foxp3 dans au moins une cellule, de préférence quand au moins 15 % de la population cellulaire présente le phénotype CD43-.

Dans un mode de réalisation, la population de cellules T obtenue selon la méthode de l'invention comprend des cellules T régulateurs (Treg), des cellules T effecteurs (Teff) et des cellules T CD4-CD8-CD3+TCRab+.

Selon un mode de réalisation, la méthode selon l'invention comprend deux étapes principales de culture cellulaire, séparées par une étape intermédiaire. La première étape principale permet d'obtenir des cellules souches hématopoïétiques à partir de cellules souches pluripotentes, préférentiellement lesdites cellules souches hématopoïétiques sont comprises dans un corps embryoïde. L'étape intermédiaire consiste à dissocier les corps embryoïdes. La seconde étape principale permet d'obtenir des cellules T comprenant des cellules T régulatrices à partir des cellules souches hématopoïétiques obtenues en étape 1, et comprend la culture desdites cellules en présence d'au moins un ligand de Notch, par exemple avec des cellules exprimant au moins un ligand de Notch. De manière concomitante, cette seconde étape de différentiation en lymphocytes T comprend également l'introduction d'un vecteur comprenant une séquence d'acide nucléique codant pour Foxp3 dans au moins une cellule.

La première étape selon l'invention permet d'obtenir des cellules souches hématopoïétiques à partir de cellules souches pluripotentes.

De préférence, ces cellules souches pluripotentes sont des cellules souches pluripotentes de mammifère. De façon préférée, ces cellules souches pluripotentes sont des cellules humaines, de préférence des cellules souches pluripotentes humaines induites ou des cellules souches embryonnaires humaines.

Selon un mode de réalisation, les cellules souches pluripotentes sont des cellules souches pluripotentes humaines induites.

Les cellules souches pluripotentes humaines induites selon l'invention peuvent être obtenues par reprogrammation de cellules différenciées, par des méthodes connues de l'art, notamment par l'introduction des gènes Oct3/4, Sox2, c-Myc, et Klf4 par des vecteurs viraux, des plasmides, des virus de Sendaï, ou par injection dans les cellules d'ARNm. Les cellules différenciées peuvent être, par exemple, des fibroblastes cutanés, des kératinocytes, des adipocytes, des cellules hématopoïétiques.

Des exemples de cellules souches pluripotentes humaines induites comprennent les cellules hiPS T04, les cellules hiPS LON80, hiPS LON71, hiPS BJ1, et hiPS MiPS.

Selon un mode de réalisation, les cellules différenciées destinées à être reprogrammées en cellules souches pluripotentes induites sont issues d'un échantillon biologique prélevé sur un sujet. L'échantillon biologique peut par exemple être choisi parmi une biopsie, un échantillon sanguin, de plasma, de sérum, de salive, ou un prélèvement urinaire.

Ainsi, selon un mode de réalisation de l'invention, le procédé de l'invention comprend une première étape d'obtention de cellules souches pluripotentes induites à partir de cellules différenciées prélevées sur un sujet.

Selon un mode de réalisation, les cellules souches pluripotentes sont des cellules souches embryonnaires humaines.

Des exemples de cellules souches embryonnaires humaines citées seulement comme référence comprennent les cellules hES WA09, les cellules hES WA01, hES WA07, hES BG01, hES et HUES7.

Selon un mode de réalisation particulier, les cellules souches pluripotentes sont des cellules souches embryonnaires qui ne sont pas obtenues par une destruction d'embryons humains. Un tel procédé pour obtenir de telles cellules souches est notamment décrit par Chung et al. (2008).

Selon un autre mode de réalisation de l'invention, les cellules souches pluripotentes ne sont pas des cellules souches embryonnaires humaines.

Avant leur différenciation en cellules souches hématopoïétiques par la méthode de l'invention, les cellules souches pluripotentes peuvent être maintenues dans un milieu de culture convenant à leur maintien pendant plusieurs jours à plusieurs semaines, et notamment pendant environ 1 à 10 jours, en général pendant environ 5 jours après le dernier passage. Des milieux convenant au maintien des cellules souches pluripotentes sont connus de l'art. Selon un mode de réalisation, le milieu de culture des cellules souches pluripotentes comprend du DMEM/F12 (milieu Eagle modifié de Dulbecco et nutriments, ThermoFisher).

De façon préférée, le milieu de culture pour cellules souches pluripotentes comprend entre environ 5 et environ 40 %, de préférence environ 20 % de sérum KSR (« KnockOut serum replacement », ThermoFisher).

Selon un mode de réalisation, le milieu de culture pour cellules souches pluripotentes comprend d'environ 0,1 % à environ 5 % de L-glutamine, de préférence environ 1 % de L-glutamine.

Selon un mode de réalisation, le milieu de culture pour cellules souches pluripotentes comprend d'environ 0,1 % à environ 5 % d'acides aminés non-essentiels, de préférence environ 1 % d'acides aminés non-essentiels.

Selon un mode de réalisation, le milieu de culture pour cellules souches pluripotentes comprend d'environ 0,01 % à environ 0,5 % de 2-mercaptoéthanol, de préférence environ 0,1 % de 2-mercaptoéthanol.

Selon un mode de réalisation, le milieu de culture pour cellules souches pluripotentes comprend d'environ 1 à environ 30 ng/mL de FGF2 (facteur de croissance de fibroblastes 2, ThermoFisher), de préférence environ 10 ng/mL de FGF2.

Selon un mode de réalisation, le milieu de culture pour cellules souches pluripotentes, comprenant de préférence du DMEM/F12, comprend du sérum KSR, de la L-glutamine, des acides aminés non-essentiels, du 2-mercaptoéthanol et/ou du FGF2 tels que décrit ci-dessus.

Selon un mode de réalisation, le milieu de culture pour cellules souches pluripotentes, comprenant de préférence du DMEM/F12, comprend du sérum KSR, de la L-glutamine, des acides aminés non-essentiels, du 2-mercaptoéthanol et du FGF2 tels que décrit ci-dessus.

Selon un mode de réalisation, les cellules souches pluripotentes sont mises en culture afin d'obtenir des cellules souches hématopoïétiques. Plusieurs conditions de culture mises en œuvre pour permettre l'apparition de cellules souches hématopoïétiques sont connues de l'art.

Ainsi, la première étape du procédé selon l'invention (correspondant à l'étape a) du procédé selon l'invention) consiste à cultiver des cellules souches pluripotentes dans un milieu de culture exempt de sérum, comprenant du BMP, du FGF2, du VEGF, du SCF, du Flt3-L et/ou de l'IL-3, dans des conditions permettant d'obtenir des corps embryoïdes comprenant au moins 5% de cellules CD34+CD43+, de préférence pendant au moins 9 jours.

Les cellules souches pluripotentes sont cultivées dans des conditions permettant d'induire la formation de corps embryoïdes. A cet effet, la mise en culture des cellules peut être effectuée, à titre illustratif, dans une plaque à faible adhérence (Sigma-Aldrich, Fisher), qui favorise l'apparition d'agrégats cellulaires en trois dimensions (corps embryoïdes) et reproduit de façon plus efficace les interactions intercellulaires existant lors du développement de l'embryon dans le corps de l'animal.

Dans un mode de réalisation, la culture des cellules souches pluripotentes à l'étape a) est réalisée dans un milieu de culture exempt de sérum, comprenant du BMP, du FGF2, du VEGF, du SCF, du Flt3-L et/ou de l'IL-3, adapté pour induire la formation de corps embryoïdes, pendant au moins 9 jours, dans des conditions permettant d'obtenir des corps embryoïdes comprenant au moins 5 % de cellules CD34+CD43+.

Les milieux de culture convenant à la croissance des cellules hématopoïétiques sont connus de l'état de l'art (voir par exemple Chang et al. (2014)).

Selon un mode de réalisation, le milieu de culture pour induire la formation de corps embryoïdes comprend un milieu de culture comprenant du BMP, du FGF2, du VEGF, du SCF, du Flt3-L et/ou de l'IL-3, convenant à la croissance des cellules hématopoïétiques exempt de sérum, par exemple le milieu StemPro-34 SFM (ThermoFisher).

Selon un mode de réalisation, le milieu de culture pour induire la formation de corps embryoïdes comprend d'environ 0,1 à environ 5 % de L-glutamine, de préférence environ 1 % de L-glutamine.

Selon un mode de réalisation, le milieu de culture pour induire la formation de corps embryoïdes comprend d'environ 0,1 à environ 5 % d'acides aminés non-essentiels, de préférence environ 1 % d'acides aminés non-essentiels.

Selon un mode de réalisation, le milieu de culture pour induire la formation de corps embryoïdes comprend d'environ 0,01 à environ 0,5 % de 2-mercaptoéthanol, de préférence environ 0,1 % de 2-mercaptoéthanol.

Selon un mode de réalisation, le milieu de culture pour induire la formation de corps embryoïdes comprend d'environ 10 à environ 1000 U/mL de pénicilline, de préférence environ 100 U/mL de pénicilline.

Selon un mode de réalisation, le milieu de culture pour induire la formation de corps embryoïdes comprend d'environ 10 à environ 1000 ng/mL de streptomycine, de préférence environ 100 ng/mL de streptomycine.

Selon un mode de réalisation, le milieu de culture pour induire la formation de corps embryoïdes comprend d'environ 5 à environ 1000 µg/mL, de préférence environ 50 µg/mL d'acide ascorbique.

Selon un mode de réalisation, milieu de culture convenant à la croissance des cellules hématopoïétiques exempt de sérum, par exemple le milieu StemPro-34 SFM, comprend de la L-glutamine, des acides aminés non-essentiels, du 2-mercaptoéthanol, de la pénicilline, de la streptomycine et/ou de l'acide ascorbique tels que décrit ci-dessus.

Dans un mode de réalisation, la culture des cellules souches pluripotentes à l'étape a) est réalisée dans un milieu de culture convenant à la croissance des cellules hématopoïétiques exempt de sérum, par exemple le milieu StemPro-34 SFM (ThermoFisher), comprenant du BMP, du FGF2, du VEGF, du SCF, du Flt3-L et/ou de l'IL-3, pendant au moins 9 jours, dans des conditions permettant d'obtenir des corps embryoïdes comprenant au moins 5 % de cellules CD34+CD43+.

Dans un mode de réalisation, la culture des cellules souches pluripotentes à l'étape a) est réalisée dans un milieu de culture convenant à la croissance des cellules hématopoïétiques exempt de sérum, par exemple le milieu StemPro-34 SFM (ThermoFisher), comprenant du BMP, du FGF2, du VEGF, du SCF, du Flt3-L et de l'IL-3, pendant au moins 9 jours, dans des conditions permettant d'obtenir des corps embryoïdes comprenant au moins 5 *%* de cellules CD34+CD43+.

Selon un mode de réalisation préféré, les cellules souches pluripotentes sont d'abord incubées en présence de BMP (*bone morphogenetic protein*) pour faciliter l'induction de la formation des corps embryoïdes. Selon un mode de réalisation préféré, les cellules souches pluripotentes sont incubées pendant 10 à 48 heures, de préférence pendant une journée, en présence de BMP. Selon un autre mode de réalisation, les cellules sont incubées en présence de 3 à 300 ng/mL de BMP, de préférence de 10 à 100 ng/mL de BMP, plus préférentiellement de 20 à 50 ng/mL de BMP, et en particulier d'environ 30 ng/mL de BMP. De préférence, la BMP est la BMP-4, de préférence la BMP-4 humaine (hBMP-4). De façon plus préférée, les cellules sont incubées pendant une journée en présence d'environ 30 ng/mL de hBMP-4a, de préférence à J0 (début de la seconde phase).

Dans un mode de réalisation, suite à l'incubation en présence de BMP, de préférence BMP-4, un mélange comprenant de la BMP (de préférence BMP-4) et du FGF2 est ajouté au milieu pour permettre l'induction de mésoderme.

De préférence, entre environ 3 ng/mL et environ 300 ng/mL de BMP, de préférence BMP-4, et entre environ 0,5 ng/mL et environ 50 ng/mL de FGF2 sont ajoutés au milieu, de préférence environ 30 ng/mL de BMP, de préférence BMP-4, et environ 5 ng/mL de FGF2 sont ajoutés au milieu. Selon un mode de réalisation, cet ajout est réalisé en une seule fois, de préférence au jour 1 de la phase de l'induction de la formation des corps embryoïdes (jour J1).

Dans un mode de réalisation, une solution comprenant des facteurs de croissance et/ou des cytokines est ajoutée tous les deux jours, de préférence à partir du jour 3 de la phase de l'induction de la formation des corps embryoïdes (jour J3), jusqu'à la fin de la phase de l'induction de la formation des corps embryoïdes.

La fin de la phase de l'induction de la formation des corps embryoïdes correspond au moment où les corps embryoïdes sont dissociés. Ceci peut par exemple se produire au jour 5, jour 6, jour 7, jour 8, jour 9, jour 10, jour 11, jour 12 de la phase de l'induction de la formation des corps embryoïdes, ou plus tard (J5, J6, J7, J8, J9, J10, J11, J12 ou plus tard).

Dans un mode de réalisation particulier, l'étape a) de culture des cellules souches pluripotentes est réalisée pendant au moins 9 jours.

Le terme « au moins 9 jours » inclut 9 jours, 10 jours, 11 jours ou plus. Dans un mode de réalisation, l'étape a) de culture des cellules souches pluripotentes est ainsi réalisée pendant 9 jours.

Dans un mode de réalisation, l'étape a) est réalisée pendant 9 à 17 jours, de préférence pendant 9 à 15 jours, préférentiellement pendant 9 à 14 jours. Dans un mode de réalisation, l'étape a) est réalisée pendant 9 à 12 jours, de préférence pendant 9 à 11 jours, préférentiellement pendant 9 à 10 jours.

Dans un mode de réalisation, la solution comprenant des facteurs de croissance et/ou cytokines comprend du VEGF (facteur de croissance de l'endothélium vasculaire, ThermoFisher), du SCF (facteur de croissance des cellules souches, ThermoFisher), FLt3-L (ligand de la tyrosine kinase « Fms-like tyrosine kinase 3 », ThermoFisher), de l'IL-3 (interleukine 3 recombinante, ThermoFisher) et/ou FGF2.

Une solution comprenant des facteurs de croissance et/ou cytokines préférée comprend du VEGF (facteur de croissance de l'endothélium vasculaire, ThermoFisher), du SCF (facteur de croissance des cellules souches, ThermoFisher), FLt3-L (ligand de la tyrosine kinase « Fms-like tyrosine kinase 3 », ThermoFisher), de l'IL-3 (interleukine 3 recombinante, ThermoFisher) et FGF2.

De façon préférée, la solution comprenant des facteurs de croissance et/ou cytokines comprend d'environ 2 ng/mL à environ 200 ng/mL VEGF, de préférence environ 20 ng/mL.

De façon préférée, la solution comprenant des facteurs de croissance et/ou des cytokines comprend d'environ 10 ng/mL à environ 300 ng/mL de SCF, de préférence environ 100 ng/mL de SCF.

De façon préférée, la solution comprenant des facteurs de croissance et/ou des cytokines comprend d'environ 2 ng/mL à environ 200 ng/mL de Flt3L, de préférence environ 20 ng/mL de Flt3L.

De façon préférée, la solution comprenant des facteurs de croissance et/ou des cytokines comprend d'environ 2 ng/mL à environ 200 ng/mL de hIL-3, de préférence environ 20 ng/ml de hIL-3.

De façon préférée, la solution comprenant des facteurs de croissance et/ou des cytokines comprend d'environ 0,5 ng/mL à environ 50 ng/mL de FGF2, de préférence environ 5 ng/mL de FGF2.

De façon préférée, une solution comprenant des facteurs de croissance et/ou des cytokines qui ne comprend pas de FGF2 est utilisée juste avant la fin de la phase de l'induction de la formation des corps embryoïdes. De préférence, cette solution est utilisée à partir du jour J7 lors de la phase de l'induction de la formation des corps embryoïdes.

Selon un mode de réalisation de l'invention, les corps embryoïdes obtenus comprennent des cellules souches hématopoïétiques, capables d'exprimer le marqueur CD34. Ainsi, les inventeurs ont montré que la première étape du procédé de l'invention permet l'obtention d'une sous-population de cellules souches hématopoïétiques présentant le phénotype CD34+. Plus particulièrement, environ 40 % de la population cellulaire totale exprime CD34+ après 7 jours de culture (première phase). Les inventeurs ont de plus mis en évidence l'obtention d'une sous-population CD34+CD43+ et d'une sous-population CD34+CD43-, ces deux populations étant présentes en proportions relativement équivalentes dans la population. De façon surprenante, les inventeurs ont montré que l'augmentation de la sous-population CD34+CD43+ dans les corps embryoïdes, et la présence de la sous-population CD34+CD43- dans les corps embryoïdes, rendent la population cellulaire totale plus apte à poursuivre le protocole de différenciation cellulaire selon l'invention.

Les inventeurs ont démontré que les conditions permettant d'obtenir des corps embryoïdes comprenant au moins 5 % de cellules CD34+CD43+ découlent de la nature du milieu de culture, de la présence des différents facteurs et du temps de culture, ces paramètres étant définis ci-dessus.

Dans le cadre de l'invention, « au moins 5% » inclut 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 et 60 %. Dans un mode de réalisation, les conditions de culture de l'étape a) permettent d'obtenir des corps embryoïdes comprenant entre environ 5 % et environ 30, 40, 50 ou 60% de cellules CD34+CD43+.

Dans un mode de réalisation, les conditions de culture de l'étape a) permettent d'obtenir des corps embryoïdes comprenant au moins 10% de cellules CD34+CD43+. Dans un mode de réalisation, les conditions de culture de l'étape a) permettent d'obtenir des corps embryoïdes comprenant au moins 15% de cellules CD34+CD43+.

Dans un mode de réalisation, les conditions de culture de l'étape a) permettent d'obtenir des corps embryoïdes comprenant entre environ 5 % et environ 60% de cellules CD34+CD43+, de préférence entre environ 10% et environ 60% de cellules CD34+CD43+, préférentiellement entre environ 15% et environ 60% de cellules CD34+CD43+.

Selon un mode de réalisation, le procédé de l'invention comprend la dissociation des corps embryoïdes (correspondant à l'étape b) du procédé de l'invention, également qualifiée d'étape intermédiaire ci-dessus), avant leur transfert dans des conditions de culture permettant la différenciation des cellules souches hématopoïétiques en cellules T.

Les corps embryoïdes peuvent être dissociés par des méthodes connues de l'art. Par exemple, les corps embryoïdes peuvent être dissociés par séparation enzymatique (trypsine, collagénase, dispase, etc.) ou par séparation mécanique.

Selon un mode de réalisation, la dissociation des corps embryoïdes est une séparation enzymatique réalisée par un traitement à l'Accutase (Invitrogen).

Selon un mode de réalisation, les corps embryoïdes sont dissociés lorsqu'au moins 5 %, de préférence au moins 10%, de la population cellulaire présente le phénotype CD34+CD43+.

Selon un mode de réalisation, les corps embryoïdes sont dissociés lorsqu'au moins 15 % de la population cellulaire présente le phénotype CD34+CD43+. De préférence, les corps embryoïdes sont dissociés lorsqu'au moins 20 % de la population cellulaire présente le phénotype CD34+CD43+.

Selon un mode de réalisation, les corps embryoïdes sont dissociés lorsqu'au moins 5, 6, 7, 8, 9, 10, 11, 12, 13, 14, 15, 16, 17, 18, 19, 20, 21, 22, 23, 24, 25, 26, 27, 28, 29 ou 30 % de la population de cellules souches hématopoïétiques présente le phénotype CD34+CD43+.

Ainsi, selon un mode de réalisation, la dissociation des corps embryoïdes est effectuée au plus tôt au jour 9 de culture pour la différenciation des cellules souches pluripotentes en corps embryoïdes (« J9 »). Selon un mode de réalisation, la dissociation des corps embryoïdes est effectuée entre le jour 9 (« J9 ») et le jour 12 (« J12 »), de préférence entre le jour 9 (« J9 ») et le jour 11 (« J11 »), de préférence entre le jour 9 (« J9 ») et le jour 10 (« J10 ») de culture pour la différenciation des cellules souches pluripotentes en corps embryoïdes. Selon un mode de réalisation, la dissociation des corps embryoïdes est effectuée au jour 9 (« J9 »), au jour 10 (« J10 »), ou au jour 11 (« J11 ») de culture pour la différenciation des cellules souches pluripotentes en corps embryoïdes. Selon un mode de réalisation, la dissociation des corps embryoïdes est effectuée au jour 9 de culture pour la différenciation des cellules souches pluripotentes en corps embryoïdes (« J9 »).

Les méthodes pour mesurer l'expression de marqueurs cellulaires sont connues de l'art.

Selon un mode de réalisation, la cytométrie en flux est utilisée pour mesurer la présence de marqueurs dans la population cellulaire.

Des anticorps pouvant être utilisés pour quantifier CD34 sont par exemple des anticorps conjugués anti-CD34, par exemple l'anticorps CD34-PE-Cy7 (Fisherscientific), ou l'anticorps CD34-APC (BD Biosciences).

Des anticorps pouvant être utilisés pour quantifier CD43 sont par exemple anticorps conjugués anti-CD43, par exemple l'anticorps CD43-APC (Fisherscientific), l'anticorps CD43eBioR2/60 (Invitrogen), ou l'anticorps CD43-FITC (Fisherscientific).

Les méthodes connues de cytométrie en flux permettent de compter les cellules d'intérêt, en les mettant en suspension dans un flux fluidique et en les passant au travers d'un appareil de détection électronique. Les méthodes de cytométrie en flux permettent des analyses multiparamétriques simultanées des paramètres physiques et/ou chimiques de plusieurs milliers de particules par seconde, comme des paramètres fluorescents. Les instruments modernes de cytométrie en flux possèdent typiquement plusieurs lasers et détecteurs de fluorescence.

La cytométrie en flux permet également de séparer physiquement les cellules en mélange hétérogène selon leurs propriétés, de manière à isoler ou purifier des populations d'intérêt. Cette méthode est connue sous le nom de « tri cellulaire à fluorescence activée » ou « FACS ». Selon cette méthode, le mélange cellulaire complexe initial est d'abord marqué avec un ou plusieurs anticorps spécifiques de marqueurs de surface cellulaire qui ont été conjugués à des colorants fluorescents. Des cellules peuvent également être analysées qui expriment une ou plusieurs protéines fluorescentes recombinantes conjointement avec un gène d'intérêt, permettant la sélection de cellules sans utiliser d'anticorps.

Une autre méthode permettant le tri cellulaire est le tri cellulaire par activation magnétique, ou « MACS ». Cette méthode met en œuvre des nanoparticules magnétiques (« billes ») enrobées d'anticorps spécifiques de certains marqueurs de surface cellulaire. Il est possible alors de choisir des anticorps spécifiques soit des marqueurs exprimés par la population d'intérêt (sélection positive), soit exprimés par types de cellules indésirables (sélection négative). Après l'incubation des nanoparticules avec la population cellulaire, la suspension est ajoutée à une colonne de séparation spéciale à usage unique fixée à un aimant auquel les billes adhèrent, tandis que les cellules non marquées s'écoulent.

Selon un mode de réalisation de l'invention, les cellules obtenues après dissociation des corps embryoïdes sont transférées dans un second milieu de culture pour la seconde étape de culture cellulaire du procédé de l'invention (correspondant à l'étape c) du procédé de l'invention). La seconde étape de culture cellulaire du procédé de l'invention permet la différenciation des cellules souches hématopoïétiques CD34+ issues des corps embryoïdes en cellules T.

Selon un mode de réalisation, l'ensemble des cellules issues de la dissociation des corps embryoïdes est transféré dans les conditions de cultures permettant la différenciation des cellules souches hématopoïétiques en cellules T.

Dans un mode de réalisation, les corps embryoïdes dissociés, obtenus à l'issue de l'étape b), sont placés en culture dans un milieu de culture de différenciation comprenant du SCF, du Flt3-L et/ou de l'IL-7, de préférence dans un milieu de culture de différenciation comprenant du SCF, du Flt3-L et de l'IL-7, de préférence pendant au moins 15 jours, avec au moins un ligand de Notch, de préférence en coculture avec des cellules exprimant au moins un ligand de Notch, de sorte à obtenir ladite population de cellules T.

Selon un mode de réalisation, les cellules souches hématopoïétiques (*i.e*., des cellules présentant le phénotype CD34+) issues des corps embryoïdes sont isolées puis placées dans les conditions de cultures permettant la différenciation des cellules souches hématopoïétiques en cellules T.

La différenciation en cellules T est réalisée en présence d'au moins un ligand de Notch, (e.g. ligands Delta-L1 ou Delta-L4), de préférence en présence d'une cellule exprimant au moins un ligand de Notch, encore plus préférentiellement en présence d'une cellule exprimant au moins un ligand de Notch (exemples : OP9-DLL1 ou OP9-DLL4 ou un mélange de celles-ci). Ces cellules sont connues de l'art. Notamment, les lignées cellulaires OP9-DLL1 et/ou OP9-DLL4 peuvent être utilisées.

Selon un mode de réalisation, le milieu de culture pour la différenciation en cellules T comprend du MEM (Milieu Essentiel Minimum, Dutscher).

Selon un mode de réalisation, le milieu de culture pour la différenciation en cellules T comprend d'environ 2 à environ 30 % de SVF (Sérum de Veau Fœtal), de préférence environ 20 % de SVF.

Selon un mode de réalisation, le milieu de culture pour la différenciation en cellules T comprend d'environ 0,1 à environ 5 % de L-glutamine, de préférence environ 1 % de L-glutamine.

Selon un mode de réalisation, le milieu de culture pour la différenciation en cellules T comprend d'environ 0,1 à environ 5 % d'acides aminés non-essentiels, de préférence environ 1 % d'acides aminés non-essentiels.

Selon un mode de réalisation, le milieu de culture pour la différenciation en cellules T comprend d'environ 0,01 à environ 0,5 % de 2-mercaptoéthanol, de préférence environ 0,1 % de 2-mercaptoéthanol.

Selon un mode de réalisation, le milieu de culture pour la différenciation en cellules T comprend d'environ 5 à environ 1000 µg/mL, de préférence environ 10 à 100 µg/mL d'acide ascorbique, plus particulièrement environ 50 µg/mL d'acide ascorbique.

Selon un mode de réalisation, le milieu de culture pour la différenciation en cellules T, de préférence comprenant du MEM (Milieu Essentiel Minimum, Dutscher), comprend du SVF, de la L-glutamine, des acides aminés non-essentiels, du 2-mercaptoéthanol et/ou de l'acide ascorbique tels que décrits ci-dessus. Dans un mode de réalisation, pendant la phase de différenciation en cellules T (seconde phase), une solution comprenant des facteurs de croissance et/ou des cytokines est ajoutée dans le milieu. La solution comprenant des facteurs de croissance et/ou cytokines comprend du SCF (facteur de croissance des cellules souches), FLt3-L (ligand de la tyrosine kinase « Fms-like tyrosine kinase 3 »), et/ou de l'IL-7 (interleukine 7).

De façon préférée, la solution comprenant des facteurs de croissance et/ou cytokines préférée comprend du SCF (facteur de croissance des cellules souches), FLt3-L (ligand de la tyrosine kinase « Fms-like tyrosine kinase 3 »), et de l'IL-7 (interleukine 7).

De façon préférée, la solution comprenant des facteurs de croissance et/ou des cytokines comprend d'environ 5 ng/mL à environ 300 ng/mL de SCF, de préférence environ 5 ng/mL à environ 50 ng/mL de SCF, plus particulièrement environ 10 ng/mL de SCF.

De façon préférée, la solution comprenant des facteurs de croissance et/ou des cytokines comprend d'environ 2 ng/mL à environ 200 ng/mL de Flt3L, de préférence environ 5 ng/mL à environ 50 ng/mL de Flt3L, plus particulièrement environ 10 ng/mL de Flt3L.

De façon préférée, la solution comprenant des facteurs de croissance et/ou des cytokines comprend d'environ 2 ng/mL à environ 200 ng/mL d'IL-7 (interleukine 7, Gibco), de préférence environ 2 ng/mL à environ 50 ng/mL d'IL-7, en particulier environ 5 ng/ml d'IL-7.

Selon un mode de réalisation, la solution comprenant des facteurs de croissance et/ou des cytokines est ajoutée dans le milieu tous les uns, deux ou trois jours, de préférence tous les deux jours, pendant la phase de différenciation en cellules T (seconde phase).

Selon un mode de réalisation, les cellules sont transférées sur un nouveau milieu de culture pour la différenciation en cellules T en présence d'au moins un ligand de Notch, par exemple en présence de cellules exprimant au moins un ligand de Notch, tous les 3, 4, 5 ou 6 jours, de préférence tous les 5 jours, à partir du début de la phase de différenciation en cellules T.

Selon un mode de réalisation, la phase de différenciation en cellules T dure au moins 7 jours, de préférence au moins 10 jours, de préférence au moins 15 jours, de préférence au moins 20 jours, de préférence au moins 25 jours. Selon un mode de réalisation, la phase de différenciation en cellules T dure environ 26 jours.

La méthode selon l'invention comprend en outre l'introduction entre le jour 8 (Jd8) et le jour 12 (Jd12) d'un vecteur comprenant une séquence d'acide nucléique codant pour Foxp3 (correspondant à l'étape d) du procédé selon l'invention). Selon un mode de réalisation, le vecteur comprend en plus la séquence d'acide nucléique d'au moins un autre gène, ou codant pour au moins un autre ARNm d'intérêt, éventuellement traduit en un polypeptide d'intérêt supplémentaire. Des exemples de telles séquences d'acide nucléique d'intérêt incluent une séquence d'acide nucléique codant pour un récepteur antigénique chimérique (CAR), une séquence d'acide nucléique codant pour Mcl-1, une séquence d'acide nucléique codant pour Bcl-xL, et une séquence d'acide nucléique codant pour Helios.

Selon un mode de réalisation, l'étape d) d'introduction d'un vecteur comprenant une séquence d'acide nucléique codant pour Foxp3 est réalisée entre le jour Jd8 et le jour Jd12, de préférence entre le jour Jd9 et le jour Jd11, de l'étape c), c'est-à-dire de la phase de différenciation en cellules T. Selon un mode de réalisation, l'étape d) d'introduction d'un vecteur comprenant une séquence d'acide nucléique codant pour Foxp3, est réalisée au jour Jd9, au jour Jd10 ou au jour Jd11 de la phase de différenciation en cellules T (seconde phase principale de culture cellulaire, correspondant à l'étape c) du procédé de l'invention).

Le terme « Jd » indique le nombre de jour de la phase de différenciation en cellules T, c'est-à-dire de la seconde phase de culture cellulaire.

De préférence, l'introduction d'un vecteur comprenant une séquence d'acide nucléique codant pour Foxp3, est réalisée le jour Jd10 de la seconde phase de culture cellulaire, c'est-à-dire pendant l'étape c) de la méthode de la présente invention. Plus préférentiellement, l'introduction d'un vecteur comprenant une séquence d'acide nucléique codant pour Foxp3, est réalisée le 10^{ème} jour suivant la mise en présence des cellules avec au moins un ligand de Notch.

En effet, de façon surprenante, les inventeurs ont montré que les phénotypes cellulaires observés au jour 26 de la seconde phase (« Jd26 ») étaient particulièrement améliorés lorsque la transduction avait été effectuée au jour Jd10 de la seconde phase de culture cellulaire. En effet, les cellules observées au jour Jd26 de la seconde phase comprennent une plus grande proportion de cellules exprimant CD3 et le TCR lorsque la transduction avait été effectuée au jour Jd10 de la seconde phase de culture cellulaire. Les inventeurs ont par ailleurs caractérisé l'expression de plusieurs marqueurs pour les cellules aux jours 8, 10 et 12 après la mise en présence d'au moins un ligand de Notch. De cette manière, ils ont mis en évidence, notamment, une diminution dans l'expression de CD43 le jour Jd10 de la seconde phase, par rapport aux jours Jd8 et Jd12. Sans vouloir être lié par une quelconque théorie, les inventeurs suggèrent qu'une augmentation de la proportion de cellules présentant le phénotype CD43- dans la population cellulaire pourrait être associée à une plus grande efficacité de l'introduction d'un vecteur comprenant une séquence d'acide nucléique codant pour Foxp3.

Ainsi, selon un mode de réalisation, l'introduction d'un vecteur comprenant une séquence d'acide nucléique codant pour Foxp3 est effectuée lorsqu'au moins 15 % de la population cellulaire présente le phénotype CD43-. De façon préférée, l'introduction d'un vecteur comprenant une séquence d'acide nucléique codant pour Foxp3 est effectuée lorsqu'au moins 20 % de la population cellulaire présente le phénotype CD43-. Plus préférentiellement, l'introduction d'un vecteur comprenant une séquence d'acide nucléique codant pour Foxp3 est effectuée lorsqu'au moins 25 % de la population cellulaire présente le phénotype CD43-.

Selon un mode de réalisation, l'introduction d'un vecteur comprenant une séquence d'acide nucléique codant pour Foxp3 est effectuée lorsque de 15 % à 40% de la population cellulaire présente le phénotype CD43-.

Il est entendu que « de 15% à 40% » inclut 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39% et 40%.

Selon un mode de réalisation de l'invention, la méthode pour obtenir une population de cellules T comprenant des cellules T régulatrices à partir de cellules souches pluripotentes, comprend les étapes suivantes :
a) cultiver des cellules souches pluripotentes dans un milieu de culture convenant à la croissance des cellules hématopoïétiques exempt de sérum, comprenant du BMP, du FGF2, du VEGF, du SCF, du Flt3-L et de l'IL-3, pendant au moins 9 jours, dans des conditions permettant d'obtenir des corps embryoïdes comprenant au moins 5 % de cellules CD34+CD43+ ;
b) dissocier lesdits corps embryoïdes ;
c) placer lesdits corps embryoïdes dissociés en culture dans un milieu de culture de différenciation comprenant du SCF, du Flt3-L, et de l'IL-7, pendant au moins 15 jours, avec au moins un ligand de Notch, de préférence en coculture avec des cellules exprimant au moins un ligand de Notch, de sorte à obtenir ladite population de cellules T ;
d) réaliser, au cours l'étape c), l'introduction entre le jour 8 (Jd8) et le jour 12 (Jd12) d'un vecteur comprenant au moins une séquence d'acide nucléique codant pour Foxp3 dans au moins une cellule, de préférence quand au moins 15 % de la population cellulaire présente le phénotype CD43-.

Selon un mode de réalisation de l'invention, le vecteur comprend une séquence d'acide nucléique codant uniquement pour Foxp3.

Selon un autre mode de réalisation, le vecteur introduit comprend en outre une autre séquence d'acide nucléique. Cette séquence supplémentaire peut par exemple correspondre au gène codant pour Mcl-1 et est décrite, par exemple, dans la demande WO2014180943. La séquence supplémentaire peut également correspondre au gène codant pour Bcl-xL tel que cela est décrit dans Haque *et al.* (2012). La séquence supplémentaire peut également correspondre au gène codant pour Helios (protéine à doigt de zinc) tel que décrit dans Baine *et al.* (2013).

Selon un mode de réalisation particulier, la séquence d'acide nucléique supplémentaire est une séquence d'acide nucléique codant pour un récepteur antigénique chimérique (CAR). Selon un mode de réalisation, le récepteur antigénique chimérique (CAR) est un CAR anti-HLA, c'est-à-dire un CAR ciblant ou reconnaissant un antigène HLA, de préférence choisi parmi le groupe consistant en HLA-A, HLA-B, HLA-C, HLA-E, HLA-F, HLA-G, HLA-DM, HLA-DO, HLA-DP, HLA-DQ, et HLA-DR. Ainsi, selon un mode de réalisation, la séquence d'acide nucléique supplémentaire est une séquence d'acide nucléique codant pour un récepteur antigénique chimérique (CAR) anti-HLA-A2 (c'est-à-dire un CAR ciblant ou reconnaissant HLA-A2), tel que cela est décrit dans MacDonald *et al.* (2016).

Les CARs sont des récepteurs artificiels constitués d'un domaine de ciblage associé à un ou plusieurs domaine(s) de signalisation intracellulaire, généralement via un domaine transmembranaire. Les CARs sont conçus pour reconnaître un antigène présent à la surface des cellules à cibler, par exemple des cellules immunitaires à réprimer dans le cadre d'un traitement anti-rejet ou des cellules tumorales dans le cadre d'un traitement anti-cancer.

En général, le domaine de ciblage d'un CAR est un fragment variable monocaténaire d'un anticorps (scFv). Des domaines de ciblage dérivés de domaines de liaison de récepteurs ou de ligands ont aussi été utilisés avec succès. Le domaine de signalisation intracellulaire des CARs de première génération est un domaine de stimulation généralement dérivé de CD3zéta. Les CARs de seconde et troisième génération comprennent en outre un (CAR de seconde génération) ou plusieurs (CAR de troisième génération) domaines de signalisation intracellulaires dérivés de molécules co-stimulatrices, telles que CD28, OX-40 (CD134), et 4-1BB (CD137).

Les stratégies pour conceptualiser et produire des récepteurs antigéniques chimériques (CARs) sont bien connues dans l'état de l'art. A titre illustratif, il peut être cité les références suivantes : Bonini and Mondino (2015), Srivastava and Riddell (2015), Jensen and Riddell (2015), et Gill and June (2015).

L'homme du métier est capable d'identifier des gènes d'intérêt qui pourraient être utilisés en combinaison avec la séquence d'acide nucléique codant pour le gène de Foxp3.

Selon un mode de réalisation, le vecteur est choisi dans le groupe comprenant les vecteurs adénovirus, plasmides, vecteurs viraux adéno-associés, les vecteurs rétroviraux, les vecteurs viraux adéno-associés hybrides, les vecteurs lentivirus, les vecteurs viraux herpes simplex, les vecteurs vaccins, ou un liposome pégylé décoré, portant des anticorps spécifiques à la cellule ciblée. Selon un mode de réalisation, le vecteur est un rétrovirus, de préférence un lentivirus.

Selon un mode de réalisation, l'expression de l'acide nucléique d'intérêt implique le système Crispr-Cas9.

A l'issue de la seconde phase de culture cellulaire de la méthode selon l'invention, la population cellulaire en culture comprend des cellules Treg. Selon un mode de réalisation de l'invention, à l'issue de la seconde phase de culture cellulaire de la méthode selon l'invention, la population cellulaire en culture comprend des cellules Treg et des cellules Teff. Selon un mode de réalisation de l'invention, à l'issue de la seconde phase de culture cellulaire de la méthode selon l'invention, la population cellulaire en culture comprend des cellules Treg, et des cellules Teff et/ou des cellules T CD4-CD8-CD3+TCRab+.

Selon un mode de réalisation de l'invention, à l'issue de la seconde phase de culture cellulaire de la méthode selon l'invention, la population cellulaire en culture comprend des cellules Treg, Teff et des cellules T CD4-CD8-CD3+TCRab+.

Selon un mode de réalisation, le procédé de l'invention comprend en outre une étape d'isolement et/ou de purification des cellules T comprenant des cellules T régulatrices, dans laquelle les cellules T comprenant des cellules T régulatrices peuvent être isolées du milieu par des techniques connues. Par exemple, les méthodes FACS ou MACS qui sont décrites précédemment permettent d'isoler et purifier une population de cellules T.

Selon un autre mode de réalisation, le procédé de l'invention comprend en outre une étape d'isolement et/ou de purification des cellules Treg. Par exemple, les méthodes FACS ou MACS qui sont décrites précédemment permettent d'isoler et purifier une population de cellules Treg, par exemple Dynabeads Regulatory CD4+/CD25+ TCell Kit (Invitrogen).

Selon un mode de réalisation, le procédé de l'invention comprend en outre une étape d'isolement et/ou de purification des cellules Teff. Par exemple, les méthodes FACS ou MACS qui sont décrites précédemment permettent d'isoler et purifier une population de cellules Teff. Pour cela, les cellules peuvent être identifiées par l'utilisation de marqueurs anti-CD4, anti-CD8, anti-TCRab, et l'expression de Foxp3. Celles qui expriment CD4 et/ou CD8 et/ou le TCR mais qui n'expriment pas Foxp3 pourront être considérées comme des cellules T effectrices.

Selon un autre mode de réalisation, le procédé de l'invention comprend en outre une étape d'isolement et/ou de purification des cellules T CD4-CD8-CD3+TCRab+. Par exemple, les méthodes FACS ou MACS qui sont décrites précédemment permettent d'isoler et purifier une population de cellules T CD4-CD8-CD3+TCRab+.

Selon un mode de réalisation, le procédé de l'invention comprend en outre une étape de transduction des cellules T comprenant des cellules T régulatrices, en particulier des cellules Treg, et des cellules Teff et/ou des cellules T CD4-CD8-CD3+TCRab+, avec une séquence d'acide nucléique codant pour un récepteur antigénique chimérique (« CAR »), par exemple un CAR anti-HLA, et plus particulièrement un CAR anti-HLA-A2, tel que décrit ci-dessus, de façon à obtenir des cellules T selon l'invention exprimant un CAR, par exemple un CAR anti-HLA, et plus particulièrement un CAR anti-HLA-A2. Cette transduction peut être effectuée selon des méthodes connues de l'art. Par exemple, la transduction peut être effectuée par transfert de gène *ex vivo* par l'utilisation d'un rétrovirus ou lentivirus comprenant une séquence d'acide nucléique codant pour le CAR.

Selon un mode de réalisation, le procédé de l'invention comprend les étapes suivantes :
a) cultiver des cellules souches pluripotentes dans un milieu de culture exempt de sérum, comprenant du BMP, du FGF2, du VEGF, du SCF, du Flt3-L et/ou de l'IL-3, dans des conditions permettant d'obtenir des corps embryoïdes comprenant au moins 5% de cellules CD34+CD43+ ;
b) dissocier lesdits corps embryoïdes ;
c) placer lesdits corps embryoïdes dissociés en culture, dans un milieu de culture comprenant du SCF, du Flt3-L et/ou de l'IL-7, en présence d'au moins un ligand de Notch, de préférence pendant au moins 15 jours ;
d) concomitamment, réaliser, au cours l'étape c), l'introduction, entre le jour 8 (Jd8) et le jour 12 (Jd12), d'un vecteur comprenant au moins une séquence d'acide nucléique codant pour Foxp3 dans au moins une cellule, de préférence quand au moins 15 % de la population cellulaire présente le phénotype CD43-.

Selon un mode de réalisation, le procédé de l'invention comprend les étapes suivantes :
a) cultiver des cellules souches pluripotentes dans un milieu de culture exempt de sérum, comprenant du BMP, du FGF2, du VEGF, du SCF, du Flt3-L et/ou de l'IL-3, dans des conditions permettant d'obtenir des corps embryoïdes comprenant au moins 5% de cellules CD34+CD43+ ;
b) dissocier lesdits corps embryoïdes ;
c) placer lesdits corps embryoïdes dissociés en culture, dans un milieu de culture comprenant du SCF, du Flt3-L et/ou de l'IL-7, en présence d'au moins un ligand de Notch, de préférence pendant au moins 15 jours ;
d) réaliser, au cours l'étape c), l'introduction, entre le jour 8 (Jd8) et le jour 12 (Jd12), préférentiellement entre le jour 9 (Jd9) et le jour 11 (Jd11), d'un vecteur comprenant au moins une séquence d'acide nucléique codant pour Foxp3 dans au moins une cellule, de préférence quand au moins 15 % de la population cellulaire présente le phénotype CD43-.

Selon un mode de réalisation préféré, l'invention concerne une méthode pour obtenir une population de cellules T comprenant des cellules T régulatrices à partir de cellules souches pluripotentes, comprenant les étapes suivantes :
a) cultiver des cellules souches pluripotentes dans un milieu de culture exempt de sérum, comprenant du BMP, du FGF2, du VEGF, du SCF, du Flt3-L et/ou de l'IL-3, dans des conditions permettant l'obtention de cellules souches hématopoïétiques, comprenant le développement de corps embryoïdes comprenant au moins 5% de cellules CD34+CD43+, puis dissocier desdits corps embryoïdes ;
b) placer lesdits corps embryoïdes dissociés comprenant les cellules souches hématopoïétiques en culture, dans un milieu de culture comprenant du SCF, du Flt3-L et/ou de l'IL-7, en présence d'au moins un ligand de Notch, de préférence pendant au moins 15 jours, par exemple en coculture avec des cellules exprimant au moins un ligand de Notch, pour permettre leur différenciation en cellules T ;
c) concomitamment réaliser, au cours l'étape b), l'introduction, entre le jour 8 (Jd8) et le jour 12 (Jd12), d'un vecteur comprenant au moins une séquence d'acide nucléique codant pour Foxp3 dans au moins une cellule ;
dans laquelle les cellules sont placées en présence d'au moins un ligand de Notch selon l'étape b) lorsqu'au moins 5 % de la population cellulaire présente le phénotype CD34+CD43+, et de préférence dans laquelle l'introduction d'un vecteur prévue à l'étape c) est réalisée au moment où au moins 15 % de la population cellulaire présente le phénotype CD43-.

Selon un autre mode de réalisation préféré, l'invention concerne une méthode pour obtenir une population de cellules T comprenant des cellules T régulatrices à partir de cellules souches pluripotentes, comprenant les étapes suivantes :
a) cultiver des cellules souches pluripotentes dans un milieu de culture exempt de sérum, comprenant du BMP, du FGF2, du VEGF, du SCF, du Flt3-L et/ou de l'IL-3, dans des conditions permettant l'obtention de cellules souches hématopoïétiques, comprenant le développement de corps embryoïdes comprenant au moins 5% de cellules CD34+CD43+, puis dissocier desdits corps embryoïdes ;
b) placer lesdits corps embryoïdes dissociés comprenant les cellules souches hématopoïétiques en culture, dans un milieu de culture comprenant du SCF, du Flt3-L et/ou de l'IL-7, en présence d'au moins un ligand de Notch, de préférence pendant au moins 15 jours, par exemple en coculture avec des cellules exprimant au moins un ligand de Notch, pour permettre leur différenciation en cellules T ;
c) réaliser, au cours l'étape b), l'introduction, entre le jour 8 (Jd8) et le jour 12 (Jd12), préférentiellement entre le jour 9 (Jd9) et le jour 11 (Jd11), d'un vecteur comprenant au moins une séquence d'acide nucléique codant pour Foxp3 dans au moins une cellule ;
dans laquelle les cellules sont placées en présence d'au moins un ligand de Notch selon l'étape b) lorsqu'au moins 5 % de la population cellulaire présente le phénotype CD34+CD43+, et de préférence dans laquelle l'introduction d'un vecteur prévue à l'étape c) est réalisée au moment où au moins 15 % de la population cellulaire présente le phénotype CD43-.

Selon un autre mode de réalisation préféré, l'invention concerne une méthode pour obtenir une population de cellules T comprenant des cellules T régulatrices à partir de cellules souches pluripotentes, comprenant les étapes suivantes :
a) cultiver des cellules souches pluripotentes dans un milieu de culture exempt de sérum, comprenant du BMP, du FGF2, du VEGF, du SCF, du Flt3-L et/ou de l'IL-3, dans des conditions permettant l'obtention de cellules souches hématopoïétiques, comprenant le développement de corps embryoïdes comprenant au moins 5% de cellules CD34+CD43+, puis dissocier desdits corps embryoïdes ;
b) placer lesdits corps embryoïdes dissociés comprenant les cellules souches hématopoïétiques en culture, dans un milieu de culture comprenant du SCF, du Flt3-L et/ou de l'IL-7, en présence d'au moins un ligand de Notch, de préférence pendant au moins 15 jours, par exemple en coculture avec des cellules exprimant au moins un ligand de Notch, pour permettre leur différenciation en cellules T ;
c) réaliser, au cours l'étape b), l'introduction, entre le jour 8 (Jd8) et le jour 12 (Jd12), d'un vecteur comprenant au moins une séquence d'acide nucléique codant pour Foxp3 dans au moins une cellule ;
caractérisée en ce que les cellules sont placées en présence d'au moins un ligand de Notch selon l'étape b) lorsqu'au moins 5 %, de préférence au moins 10%, préférentiellement au moins 15%, de la population cellulaire présente le phénotype CD34+CD43+, et en ce que l'introduction d'un vecteur prévue à l'étape c) est réalisée au moment où au moins 15%, de préférence au moins 20 %, de la population cellulaire présente le phénotype CD43-.

Selon un mode de réalisation, la méthode selon l'invention permet d'obtenir une population de cellules T comprenant des Treg, et (des Teff, et/ou des cellules T CD4-CD8-CD3+TCRab+).

Selon un mode de réalisation, la méthode selon l'invention permet d'obtenir une population de cellules T comprenant des Treg CD8+CD3+TCRab+Foxp3+ et/ou CD4+CD3+TCRab+Foxp3+, des cellules T effectrices CD8+ et/ou CD4+, et des cellules T CD4-CD8-CD3+TCRab+.

Selon un mode de réalisation, la méthode selon l'invention permet d'obtenir une population de cellules T comprenant des Treg CD8+CD3+TCRab+Foxp3+ et CD4+CD3+TCRab+Foxp3+, des cellules T effectrices CD8+ et CD4+, et des cellules T CD4-CD8-CD3+TCRab+.

Selon un mode de réalisation, la population de cellules T obtenue comprend des Treg CD8+CD3+TCRab+Foxp3+ et/ou CD4+CD3+TCRab+Foxp3+, des cellules T effectrices CD8+ et/ou CD4+, et des cellules T CD4-CD8-CD3+TCRab+.

Selon un mode de réalisation, la population de cellules T obtenue comprend des Treg CD8+CD3+TCRab+Foxp3+ et CD4+CD3+TCRab+Foxp3+, des cellules T effectrices CD8+ et CD4+, et des cellules T CD4-CD8-CD3+TCRab+.

Des méthodes telles que la FACS ou MACS permettent d'isoler certaines populations particulières comme la population composée des cellules Treg, la population composée des cellules Teff, ou la population composée des cellules T CD4-CD8-CD3+TCRab+, ou leurs combinaisons. De telles méthodes permettent également d'isoler des sous populations telles que la sous population des cellules Treg CD8+CD3+TCRab+Foxp3+, la sous-population composée des cellules Treg CD4+CD3+TCRab+Foxp3+, la sous-population composée des cellules T effectrices CD8+, la sous-population composée des cellules T effectrices CD4+ ou la sous-population composée des cellules T CD4-CD8-CD3+TCRab+.

L'homme du métier est capable d'identifier et d'appliquer d'autres méthodes connues de l'état de l'art permettant d'isoler de telles populations et sous-populations cellulaires.

Selon un mode de réalisation, la population obtenue ou susceptible d'être obtenue par la méthode de l'invention est une population Treg, présentant le phénotype suivant : CD8+CD3+TCRab+Foxp3+ et/ou CD4+CD3+TCRab+Foxp3+. Selon un mode de réalisation, la population obtenue ou susceptible d'être obtenue par la méthode de l'invention se compose de Treg, et (de Teff, et/ou de cellules T CD4-CD8-CD3+TCRab+).

Selon un mode de réalisation, les cellules T susceptibles d'être obtenues par la méthode selon l'invention sont ensuite transformées (ou transduites) pour permettre l'expression d'un récepteur antigénique chimérique (« CAR »), par exemple un CAR anti-HLA, et plus particulièrement un CAR anti-HLA-A2, tel que décrit ci-dessus. Cette transformation (ou transduction) peut être effectuée selon des méthodes connues de l'art. Par exemple, la transformation (ou transduction) peut être effectuée par transfert de gène *ex vivo* par l'utilisation d'un rétrovirus ou lentivirus comprenant une séquence d'acide nucléique codant pour le CAR.

Selon un autre mode de réalisation, les cellules T susceptibles d'être obtenues par la méthode selon l'invention sont ensuite amplifiées. Par exemple, les cellules T peuvent être amplifiées par l'induction de leur prolifération de manière polyclonale en présence d'anticorps monoclonaux anti-CD3 et anti-CD28. Des méthodes d'expansion des cellules T sont connues de l'art, par exemple dans Petersen (Petersen et al. 2018 "Improving T-Cell Expansion and Function for Adoptive T-Cell Therapy Using Ex Vivo Treatment with PI3Kδ Inhibitors and VIP Antagonists." Blood Advances 2.3: 210-223) ou Boursier (Boursier et al. 2012 « Utilisation des lymphocytes T régulateurs en thérapies cellulaires dans les maladies auto-immunes ». Médecine/sciences 28 :757-63).

La population de cellules T telle que décrite ci-dessus peut être utilisée comme médicament.

Ainsi, la population de cellules T telle que décrite ci-dessus peut être utilisée en thérapie cellulaire. Particulièrement, la population de cellules T peut être utilisée en médecine personnalisée, par exemple des cellules différenciées peuvent être prélevées chez un patient, puis reprogrammées en cellules souches pluripotentes humaines induites et utilisées selon la méthode de l'invention pour fournir de nouvelles cellules T, qui pourront être administrées au dit patient.

Ainsi, la population de cellules T telle que décrite ci-dessus peut être utilisée dans le traitement d'une pathologie en lien avec un dérèglement immun.

Une pathologie en lien avec le dérèglement immun comprend les pathologies dans lesquelles la réponse immunitaire est détériorée (*e.g.,* elle ne se produit pas ou est réduite) et ne permet pas de lutter efficacement contre le pathogène, et les pathologies dans lesquelles la réponse immunitaire est trop forte et provoque par exemple des lésions aux cellules du soi. De telles pathologies correspondent, entre autres, aux infections, aux maladies auto-immunes, aux maladies inflammatoires, aux cancers, aux allergies, au rejet de greffe, ou à la réaction du greffon contre l'hôte.

Une quantité thérapeutiquement efficace de cellules T telle que décrite ci-dessus est à administrer au sujet à traiter.

Une quantité thérapeutiquement efficace de cellules T telle que décrite ci-dessus inclut, par exemple, une quantité qui est efficace pour rétablir l'homéostasie immunitaire, *i.e.* réduire l'intensité de la réponse immunitaire lorsque celle-ci est trop forte, ou accroître l'amplitude de la réponse immunitaire lorsque celle-ci est trop faible ou nulle.

La population de cellules T peut être une population de cellules Treg. La population de cellules T peut être une population de cellules Treg et peut être utilisée dans le traitement des rejets de greffe, la réaction du greffon contre l'hôte, les maladies auto-immunes et les maladies inflammatoires. La population de cellules T peut être une population de cellules Treg exprimant un récepteur antigénique chimérique (CAR), par exemple un CAR anti-HLA, et plus particulièrement un CAR anti-HLA-A2, et peut être utilisée dans le traitement anti-rejet ou le traitement de la réaction du greffon contre l'hôte.

Une population de cellules T telle que décrite ci-dessus, de préférence obtenue par la méthode de l'invention telle que décrite ci-dessus, peut être utilisée dans une méthode de modulation du système immunitaire par thérapie cellulaire.

Une population de cellules T telle que décrite ci-dessus, de préférence obtenue par la méthode de l'invention telle que décrite ci-dessus, peut être utilisée dans une méthode de traitement d'une pathologie en lien avec un dérèglement immun.

Une population de cellules Treg telle que décrite ci-dessus peut être utilisée dans une méthode de traitement d'un rejet de greffe, de la réaction du greffon contre l'hôte, d'une maladie auto-immune ou d'une maladie inflammatoire. Les cellules Treg telle que décrite ci-dessus peuvent exprimer un récepteur antigénique chimérique (CAR), par exemple un CAR anti-HLA, et plus particulièrement un CAR anti-HLA-A2. Une population de cellules Treg telle que décrite ci-dessus et exprimant un récepteur antigénique chimérique (CAR), par exemple un CAR anti-HLA, et plus particulièrement un CAR anti-HLA-A2, peut être utilisée dans une méthode de traitement d'un rejet de greffe ou de la réaction du greffon contre l'hôte.

Un kit pour la mise en œuvre de la méthode selon l'invention, peut comprendre une plaque de culture pour la formation de corps embryoïdes comprenant des cellules souches hématopoïétiques, un milieu de culture pour induire la formation de corps embryoïdes, un plaque de culture pour la différenciation des cellules souches hématopoïétiques en cellules T, un milieu de culture pour la différenciation en cellules T et des solutions comprenant des facteurs de croissance et/ou des cytokines, tels que définis ci-dessus.

### EXEMPLES

### EXEMPLE 1 DE REFERENCE

### Protocole expérimental

Le protocole de recherche a été conduit dans le respect des directives légales françaises et le comité d'éthique des institutions locales.

### Cellules souches pluripotentes

Les colonies indifférenciées de cellules souches pluripotentes humaines (lignée commerciale hES WA09 (WiCell)) ont été maintenues en culture sur fibroblastes embryonnaires de souris (« MEF ») dans un milieu pour cellules souches pluripotentes comprenant du DMEM/F12, 20 % de sérum KSR (« *KnockOut serum replacement* »), 1 % de L-glutamine, 1 % d'acides aminés non-essentiels, 0,1 % de 2-mercaptoéthanol, 10 ng/mL de FGF2 (facteur de croissance de fibroblastes) (**Figure 1****,** « Phase 0 »). Un passage est effectué tous les 5 à 6 jours.

### Méthode d'obtention de cellules souches hématopoïétiques (Phase I)

Pour la différenciation des cellules souches pluripotentes humaines en cellules hématopoïétiques, les colonies indifférenciées ont été traitées avec de la dispase (1 U/mL) pendant 10 minutes et transférées en plaques à faible adhérence pour permettre la formation de corps embryoïdes.

Le milieu de culture utilisé pour cette étape (**Figure 1****,** « phase I ») se compose de STemPro-34 (ThermoFisher), 1 % de L-glutamine, 1 % d'acides aminés non-essentiels, 0,1 % de 2-mercaptoéthanol, 100 U/mL de pénicilline et 100 ng/mL de streptomycine et 50 µg/mL d'acide ascorbique.

La formation des corps embryoïdes a été facilitée par une incubation d'une journée en présence de 30 ng/mL de hBMP-4 (« *human bone morphogenetic protein 4* ») (**figure 1****,** lettre « a »).

Les corps embryoïdes ont alors été cultivés avec 30 ng/mL de BMP-4 et 5 ng/mL de FGF2 jusqu'au jour J3 (**figure 1****,** lettre « **b** ») pour permettre l'induction du mésoderme.

Entre les jours J3 et J5, puis J5 et J7, la spéciation et la multiplication continue en présence de hVEGF (20 ng/mL) et un cocktail de facteurs de croissances et de cytokines hématopoïétiques (hSCF à 100 ng/mL, hFlt3-L à 20 ng/mL, hIL-3 à 20 ng/mL et FGF2 à 5 ng/mL) (**figure 1****,** lettre « c »).

Ensuite, la différenciation est poursuivie de J7 à J9 avec les mêmes cytokines mais sans FGF2 (**figure 1****,** lettre « d »).

### Détermination des phénotypes des cellules des corps embryoïdes à J7, J8 et J9

Les corps embryoïdes à J7, J8 et J9, contenant les cellules souches hématopoïétiques, ont été dissociés par traitement avec de l'Accutase^{®} pendant 10 minutes et les marqueurs présents ont été analysés par cytométrie en flux.

### Méthode d'obtention de cellules T (Phase II)

Les corps embryoïdes obtenus à J9 ont été dissociés par traitement avec de l'Accutase^{®} pendant 10 minutes. Les cellules ainsi isolées ont été ensemencées sur des monocouches de cellules OP9-DLL1 pour permettre leur différenciation en lymphoïdes T. Le milieu de culture « milieu OP9 » (**Figure 1****,** « phase II ») permettant cette différenciation comprend : α-MEM avec 20 % FBS, 1 % de L-glutamine, 1 % d'acides aminés non-essentiels, 0,1 % de 2-mercaptoéthanol, 100 U/mL de pénicilline, 100 ng/mL de streptomycine et 50 µg/mL d'acide ascorbique, suppléé en SCF (10 ng/mL), IL-7 (5 ng/mL) et Flt3L (5 ng/mL). Tous les deux jours, la moitié du milieu a été remplacée et tous les 5 jours, les cellules ont été transférées sur de nouvelles monocouches d'OP9-DLL1 (**Figure 1****,** lettre « e »).

### Production de lentivirus et titration

Un plasmide d'ADN a été créé qui comprend 9 µg de lentivirus portant la construction pMSCV- Human Foxp3-EF1-GFP-T2A-Puro (FOXP3GFP), 8 µg de plasmide d'empaquetage psPAX2 et 4 µg de plasmide pMD2G. Il a été transfecté dans les cellules HEK293T en utilisant la méthode au chlorure de calcium. Les particules lentivirales ont été collectées entre 42 et 66 heures après transfection dans le surnageant des cellules HEK293T. Pour la titration, 100 000 cellules de Jurkat ont été mises en cultures et contaminées avec une dilution en cascade de la moitié des virus allant de 10 µL à 0,078 µL. Trois jours après, les cellules ont été récoltées et la fluorescence de la GFP *(Green Fluorescent Protein)* a été analysée par cytométrie en flux.

### Transduction des cellules en cours de différenciation

Les cellules de Jd0, Jd5, Jd10 et Jd15 (respectivement au 5^{ème}, 10^{ème} et 15^{ème} jour après la mise en culture sur les monocouches OP9-DLL1) ont été récoltées, centrifugées et transduites avec le lentivirus FOXP3GFP à une multiplicité d'infection (MOI) de 20 particules virales par cellule dans 500 µL de milieu de culture OP9 avec les cytokines pendant 40 minutes à 37°C. Après la transduction, les cellules ont été ensemencées sur de nouvelles monocouches OP9-DLL1 dans 2 mL de milieu OP9 comprenant les cytokines.

### Cytométrie en flux

Les anticorps conjugués suivants ont été utilisés pour le phénotypage et l'analyse par cytométrie en flux : CD34-PeCY7, CD43-APC, KDR(CD309)-PE, CD7-PeCy5, CD5-BV510, CD3-APCCy7, TCRab-APC, CD4-BV605, CD8a-PE and CD8b-PeCy7 (ThermoFisher). Tous les anticorps ont été utilisés avec une dilution au vingtième. Les cellules mortes ont été exclues de l'analyse dans toutes les expérimentations par marquage au DAPI. La fluorescence a été mesurée avec un cytomètre LSR II ou Canto II (BD Biosciences) et analysée avec le logiciel FLOWJO (FlowJo LLC, Ashland, USA).

### Séquençage d'ARN « DGE-ARN »

Le kit RNeasy-Micro (Qiagen) a été utilisé pour isoler l'ARN total qui a été ensuite utilisé pour procéder au séquençage d'ARN. Le protocole pour le séquençage d'ARN 3'DGE est celui décrit dans Picarda et al. (2017).

### Résultats

La **figure 2** montre qu'après 7 jours de culture de corps embryoïdes (étape 1 du procédé de l'invention), environ 40 % des cellules expriment CD34+. Entre le jour 7 et le jour 9, le pourcentage de cellules CD34+ dans la population reste stable, et le pourcentage de CD43+ augmente. Au 9^{ème} jour de développement de corps embryoïdes, les cellules CD34+ expriment de plus hauts niveaux de facteurs de transcription ou des molécules clefs pour la différenciation lymphoïde telles que Runx3, Ikaros, et IL-7R (résultats non présentés).

L'analyse par séquençage DGERNA-Seq a montré que les cellules à J9 exprimaient Notch2. L'expression de Notch1, Notch2NL, Notch3 et Notch4 était plus élevée dans les cellules CD34+CD43- que dans les cellules CD34+CD43+ (résultats non présentés), suggérant que ces cellules CD34+CD43- étaient plus capable de se différencier, lors de la seconde phase, en coculture avec les cellules OP9-DLL1 grâce à l'expression des molécules Notch et la présence du ligand de Notch DLL1 dans le milieu de culture.

L'augmentation de la sous-population CD34+CD43+ et la présence de la sous-population CD34+CD43- dans les corps embryoïdes rendent la population cellulaire totale plus apte à se différencier lors de la seconde phase de culture cellulaire.

La **figure 3A** présente l'expression de CD5 et CD7, marqueurs de la lignée lymphoïde, par les cellules à Jd20 (20^{ème} jour de coculture avec les cellules OP9). Environ 80 % des cellules sont de phénotype CD5+CD7+ ; et parmi elles, environ 22 % sont CD4+CD8a+ (**figure 3B**), 25 % sont CD8a+CD8b+ (**figure 3C**), et 11 % sont CD56+CD8a+ (**figure 3D**).

La **figure 4A** présente quant à elle l'expression de CD5 et CD7 par les cellules à Jd26 (26^{ème} jour de coculture avec les cellules OP9). Environ 80 % des cellules sont de phénotype CD5+CD7+ ; et parmi elles, environ 22 % sont CD4+CD8a+ (**figure 4B**), 27 % sont CD8a+CD8b+ (**figure 4C**), et 11 % sont CD56+CD8a+ (**figure 4D**). Moins de 1 % de la population exprime le TCRab et CD3 (**figure 4E**). De plus, un séquençage DGE-ARN a montré que les cellules n'exprimaient pas Foxp3, le facteur de transcription clef pour les cellules Treg.

Ainsi, l'introduction du gène codant pour Foxp3 a été réalisée pour permettre aux cellules de se différencier en cellules Treg. L'insertion du gène codant pour Foxp3 a été réalisée par transduction d'un lentivirus. Différents moments ont été choisis pour effectuer la transduction des cellules, à partir de leur mise en coculture avec les cellules OP9 : Jd0 (moment de la mise en coculture), Jd5 (5 jours après la mise en coculture), Jd10 (10 jours après la mise en coculture) et Jd15 (15 jours après la mise en coculture), puis analysées à Jd26. La **figure 5** montre que des cellules transduites ont été observées pour chacune de ces transductions. Il apparaît que les transductions effectuées à Jd10 (**figure 5C**) résultent en l'obtention d'un plus grand nombre de cellules exprimant Foxp3, avec environ 11 % de la population CD7+Foxp3+.

Les marqueurs de cette sous-population de cellules CD7+Foxp3+ sont observés à Jd26 pour chaque temps de transduction. Les populations qui ont fait l'objet d'une transduction à Jd10 comprennent environ 22 % de cellules CD7+Foxp3+ exprimant CD3 et le TCR (**figure 6C**), tandis que les populations qui ont été transduites à Jd0, Jd5, Jd15 comprennent moins de 5 % (respectivement, **figures 6A****, B** et **D**). La moitié des cellules doubles positives transduites à Jd10 expriment CD8a (**figure 6E**) et l'autre moitié CD4 (**figure 6F**). Pratiquement toutes les cellules exprimant CD8a expriment aussi CD8b (**figure 6E**). Ainsi, les inventeurs sont parvenus à obtenir des cellules Treg ayant pour phénotype Foxp3+CD3+TCRab+CD8+ et Foxp3+CD3+TCRab+CD4+.

Les marqueurs de la sous-population de cellules CD7+Foxp3- (**figure 5**) sont observés à Jd26 pour chaque temps de transduction. Les populations qui ont fait l'objet d'une transduction à Jd10 comprennent environ 12 % de cellules CD7+Foxp3- exprimant CD3 et le TCR (**figure 7C**), tandis que les populations qui ont été transduites à Jd0, Jd5, Jd15 en comprennent entre 5 et 7 % (respectivement, **figures 7A****, B** et **D**). Environ 35 % de ces cellules doubles positives expriment CD8a (**figure 7E**), 38 % expriment CD4 (**figure 7F**), et environ un quart sont double négatives CD4-CD8-. Environ 70% des cellules CD8a+ expriment aussi CD8b (**figure 7E**). Ces cellules seraient des cellules T effectrices.

Pour mieux comprendre la raison pour laquelle les cellules sont plus réceptives à la transduction à Jd10, leur phénotype a été analysé en cytométrie en flux. Les résultats sont présentés en **figure 8**. Entre Jd8 et Jd12, les cellules perdent l'expression de CD34, un marqueur des cellules souches hématopoïétiques, ce qui reflète leur passage vers le lignage lymphoïde. Elles acquièrent le marqueur CD7, qui est synonyme de lignée lymphoïde précoce, et elles expriment aussi CD43 et CD38, qui sont les marqueurs des cellules immunitaires. A ce stade, les cellules n'expriment pas encore CD4 ou CD8. Il est possible de noter qu'à Jd10, environ 30 % de la population devient CD43- (**figure 8E**), alors qu'à Jd8 et Jd12, ce pourcentage n'est que d'environ 12 et 9 % respectivement (**figures 8D** et **F**). Aussi, c'est à Jd10 que la population comprend la plus grande proportion de cellules CD7+et CD43+ (environ 35 %, **figure 8E**).

### EXEMPLE 2 DE REFERENCE

Le protocole d'obtention de cellules souches hématopoïétiques (Phase I) décrit dans l'exemple 1 à partir des cellules souches commerciales hES WA09 (WiCell) été mis en œuvre à partir des lignées de cellules hES WA01 (WiCell), hiPS T04 (CRTI/PFiPSC Nantes), hiPS LON80 (PFiPSC Nantes).

La **figure 9A** confirme que les cellules hES WA09 peuvent être différenciées en cellules souches hématopoïétiques CD34+ (environ 36% à J9), et montre que la proportion de cellules CD34+CD43+ atteint environ 16,5%. La **figure 9B** montre que les cellules hiPS T04 peuvent être différenciées en cellules souches hématopoïétiques CD34+ (environ 40% à J9), et que la proportion de cellules CD34+ CD43+ atteint environ 21,5%. Les figures **9C** et **9D** montrent que les cellules hiPS LON80 et hES WA01 peuvent être différenciées en cellules souches hématopoïétiques CD34+ et que la proportion de cellules CD34+ CD43+ atteint environ 6,5% et 7,0%, respectivement.

### Bibliographie

Baine I, Basu S, Ames R, Sellers R, Macian F. (2013) Helios induces epigenetic silencing of Il2 gene expression in regulatory T cells. J Immunol. 190(3): 1008-1016.
Bonini C, Mondino A. (2015) Adoptive T-cell therapy for cancer: The era of engineered T cells. Eur J Immunol. 45(9):2457-69.
Cavazzana-Calvo Marina, Six Emmanuelle, André-Schmutz Isabelle, Coulombel Laure. (2007) Hématopoïèse humaine : des cellules CD34 aux lymphocytes T. Med Sci:23 (2) : 151-160.
Chang CW, Lai YS, Lamb LS Jr, Townes TM. (2014) Broad T-cell receptor repertoire in T-lymphocytes derived from human induced pluripotent stem cells. PLoS One. 9(5):e97335.
Chung Y, Klimanskaya I, Becker S, Li T, Maserati M, Lu SJ, Zdravkovic T, Ilic D, Genbacev O, Fisher S, Krtolica A, Lanza R. (2008) Human embryonic stem cell lines generated without embryo destruction. Cell Stem Cell. 2(2):113-7.
Gill S, June CH. (2015) Going viral: chimeric antigen receptor T-cell therapy for hematological malignancies. Immunol Rev. 263(1):68-89.
Haque Rizwanul, Lei Fengyang, Xiong Xiaofang, et al. (2012) Programming of regulatory T cells from pluripotent stem cells and prevention of autoimmunity. The Journal of Immunology, 189(3):1228-1236.
Haque, Mohammad et al. (2016) "Development of Stem Cell-Derived Antigen-Specific Regulatory T Cells Against Autoimmunity." Journal of visualized experiments : JoVE 117: 10.3791/54720.
Holmes R, Zúñiga-Pflücker JC. (2009) The OP9-DL1 system: generation of T-lymphocytes from embryonic or hematopoietic stem cells in vitro. Cold Spring Harb Protoc. Feb;2009(2):pdb.prot5156.
Jensen MC, Riddell SR. (2015) Designing chimeric antigen receptors to effectively and safely target tumors. Curr Opin Immunol. 33:9-15.
Kato Shingo, Jay A Berzofsky, Masaki Terabe. (2018) "Possible Therapeutic Application of Targeting Type II Natural Killer T Cell-Mediated Suppression of Tumor Immunity." Frontiers in Immunology 9: 314.
Lei F, Haque R, Weiler L, Vrana KE, Song J. (2009) T lineage differentiation from induced pluripotent stem cells. Cell Immunol. 260(1):1-5.
MacDonald KG, Hoeppli RE, Huang Q, Gillies J, Luciani DS, Orban PC, Broady R, Levings MK. (2016) Alloantigen-specific regulatory T cells generated with a chimeric antigen receptor. J Clin Invest. 126(4):1413-24
Passerini, Laura, Rosa Bacchetta. (2017) "Forkhead-Box-P3 Gene Transfer in Human CD4+ T Conventional Cells for the Generation of Stable and Efficient Regulatory T Cells, Suitable for Immune Modulatory Therapy." Frontiers in Immunology 8: 1282.
Picarda E, Bézie S, Boucault L, Autrusseau E, Kilens S, Meistermann D, Martinet B, Daguin V, Donnart A, Charpentier E, David L, Anegon I, Guillonneau C. (2017) Transient antibody targeting of CD45RC induces transplant tolerance and potent antigen-specific regulatory T cells. JCI Insight. 2(3):e90088.
Srivastava S, Riddell SR. (2015) Engineering CAR-T cells: Design concepts. Trends Immunol. 36(8):494-502.

## Revendications

1. Méthode pour obtenir une population de cellules T comprenant des cellules T régulatrices (Treg) à partir de cellules souches pluripotentes, comprenant les étapes suivantes :
a) cultiver des cellules souches pluripotentes dans un milieu de culture exempt de sérum, comprenant du BMP, du FGF2, du VEGF, du SCF, du Flt3-L et/ou de l'IL-3, dans des conditions permettant d'obtenir des corps embryoïdes comprenant au moins 5% de cellules CD34+CD43+ ;
b) dissocier lesdits corps embryoïdes ;
c) placer lesdits corps embryoïdes dissociés en culture, dans un milieu de culture comprenant du SCF, du Flt3-L et/ou de l'IL-7, avec au moins un ligand de Notch, de préférence en coculture avec des cellules exprimant au moins un ligand de Notch ;
d) réaliser, au cours de l'étape c), l'introduction entre le jour 8 (Jd8) et le jour 12 (Jd12) d'un vecteur comprenant au moins une séquence d'acide nucléique codant pour Foxp3 dans au moins une cellule, de préférence quand au moins 15% de la population cellulaire présente le phénotype CD43-.

2. Méthode selon la revendication **1,** dans laquelle les corps embryoïdes comprennent au moins 15% de cellules CD34+CD43+.

3. Méthode selon l'une quelconque des revendications précédentes, dans laquelle l'étape a) est réalisée pendant au moins 9 jours, de préférence pendant 9 à 12 jours.

4. Méthode selon l'une quelconque des revendications précédentes, dans laquelle les corps embryoïdes dissociés, obtenus à l'issue de l'étape b), sont placés en culture pendant au moins 15 jours.

5. Méthode selon l'une quelconque des revendications précédentes, dans laquelle le vecteur de l'étape d) est un rétrovirus, de préférence un lentivirus.

6. Méthode selon l'une quelconque des revendications précédentes, dans laquelle la séquence d'acide nucléique du vecteur de l'étape d) comprend une séquence d'acide nucléique d'intérêt supplémentaire.

7. Méthode selon l'une quelconque des revendications précédentes, dans laquelle l'acide nucléique d'intérêt supplémentaire est choisi parmi un acide nucléique codant pour un récepteur antigénique chimérique (CAR), pour Mcl-1, pour Bcl-xL, et pour Helios.

8. Méthode selon l'une quelconque des revendications précédentes, dans laquelle les cellules souches pluripotentes sont des cellules souches pluripotentes induites humaines ou des cellules souches embryonnaires humaines.

9. Méthode selon l'une quelconque des revendications précédentes, comprenant une étape supplémentaire d'isolement d'une population de cellules choisie parmi les cellules T régulatrices (Treg), les lymphocytes T effecteurs (Teff), et les lymphocytes T CD4-CD8-CD3+TCRab+.

10. Méthode selon l'une quelconque des revendications précédentes, dans laquelle la population de cellules T obtenue selon la méthode comprend des cellules T régulatrices (Treg), des cellules T effectrices (Teff) et des cellules T CD4-CD8-CD3+TCRab+.

11. Méthode selon la revendication **10,** dans laquelle la population de cellules T obtenue selon la méthode comprend des cellules Treg CD8+CD3+TCRab+Foxp3+ et CD4+CD3+TCRab+Foxp3+, des cellules T effectrices CD8+ et CD4+, et des cellules T CD4-CD8-CD3+TCRab+.

## Patentansprüche

1. Verfahren zur Gewinnung einer T-Zellpopulation, die regulatorische T-Zellen (Treg) umfasst, aus pluripotenten Stammzellen, umfassend die folgenden Schritte:
a) Kultivieren von pluripotenten Stammzellen in einem serumfreien Kulturmedium, umfassend BMP, FGF2, VEGF, SCF, Flt3-L und/oder IL-3, unter Bedingungen, die das Erhalten von Embryoidkörpern ermöglichen, die mindestens 5 % CD34+CD43+-Zellen umfassen;
b) Dissoziieren der Embryoidkörper;
c) Kultivieren der dissoziierten Embryoidkörper in einem Kulturmedium, umfassend SCF, Flt3-L und/oder IL-7, mit mindestens einem Notch-Ligand, vorzugsweise in Kokultur mit Zellen, die mindestens einen Notch-Ligand exprimieren;
d) Durchführen, während Schritt c), der Einführung zwischen Tag 8 (Jd8) und Tag 12 (Jd12) eines Vektors, umfassend mindestens eine für Foxp3 kodierende Nukleinsäuresequenz, in mindestens eine Zelle, vorzugsweise wenn mindestens 15 % der Zellpopulation den Phänotyp CD43- aufweist.

2. Das Verfahren nach Anspruch 1, wobei die Embryoidkörper mindestens 15 % CD34+CD43+-Zellen umfassen.

3. Das Verfahren nach einem der vorhergehenden Ansprüche, wobei Schritt a) für mindestens 9 Tage, vorzugsweise für 9 bis 12 Tage, durchgeführt wird.

4. Das Verfahren nach einem der vorhergehenden Ansprüche, wobei die am Ende von Schritt b) erhaltenen dissoziierten Embryoidkörper für mindestens 15 Tage kultiviert werden.

5. Das Verfahren nach einem der vorhergehenden Ansprüche, wobei der Vektor von Schritt d) ein Retrovirus ist, vorzugsweise ein Lentivirus.

6. Das Verfahren nach einem der vorhergehenden Ansprüche, wobei die Nukleinsäuresequenz des Vektors von Schritt d) eine zusätzliche Nukleinsäuresequenz von Interesse umfasst.

7. Das Verfahren nach einem der vorhergehenden Ansprüche, wobei die zusätzliche Nukleinsäure von Interesse ausgewählt ist aus einer zusätzliche Nukleinsäure für einen chimären Antigenrezeptor (CAR) kodierenden Nukleinsäure, für Mcl-1, für Bcl-xL und für Helios.

8. Das Verfahren nach einem der vorhergehenden Ansprüche, wobei die pluripotenten Stammzellen induzierte pluripotente Stammzellen des Menschen oder embryonale Stammzellen des Menschen sind.

9. Das Verfahren nach einem der vorhergehenden Ansprüche, umfassend einen zusätzlichen Schritt der Isolierung einer Zellpopulation, die ausgewählt ist aus regulatorischen T-Zellen (Treg), Effektor-T-Lymphozyten (Teff) und CD4-CD8-CD3+TCRab+ T-Lymphozyten.

10. Das Verfahren nach einem der vorhergehenden Ansprüche, wobei die nach dem Verfahren erhaltene T-Zellpopulation regulatorische T-Zellen (Treg), Effektor-T-Zellen (Teff) und CD4-CD8-CD3+TCRab+ T-Zellen umfasst.

11. Das Verfahren nach Anspruch 10, wobei die nach dem Verfahren erhaltene T-Zellpopulation CD8+CD3+TCRab+Foxp3+ und CD4+CD3+TCRab+Foxp3+ Treg-Zellen, CD8+ und CD4+ Effektor-T-Zellen und CD4-CD8-CD3+TCRab+ T-Zellen umfasst.

## Claims

1. Method for obtaining a population of T cells comprising regulatory T cells (Treg) from pluripotent stem cells, comprising the following steps:
a) culturing pluripotent stem cells in a serum-free culture medium, comprising BMP, FGF2, VEGF, SCF, Flt3-L and/or IL-3, under conditions allowing to obtain embryoid bodies comprising at least 5% of CD34+CD43+ cells;
b) dissociating said embryoid bodies;
c) placing said dissociated embryoid bodies in culture, in a culture medium comprising SCF, Flt3-L and/or IL-7, with at least one Notch ligand, preferably in coculture with cells expressing at least one Notch ligand;
d) carrying out, during step c), the introduction between day 8 (Jd8) and day 12 (Jd12) of a vector comprising at least one nucleic acid sequence encoding Foxp3 into at least one cell, preferably when at least 15% of the cell population exhibits the CD43- phenotype.

2. The method according to claim 1, wherein the embryoid bodies comprise at least 15% of CD34+CD43+ cells.

3. The method according to any one of the preceding claims, wherein step a) is carried out for at least 9 days, preferably for 9 to 12 days.

4. The method according to any one of the preceding claims, wherein the dissociated embryoid bodies, obtained at the end of step b), are placed in culture for at least 15 days.

5. The method according to any one of the preceding claims, wherein the vector of step d) is a retrovirus, preferably a lentivirus.

6. The method according to any one of the preceding claims, wherein the nucleic acid sequence of the vector of step d) comprises a further nucleic acid sequence of interest.

7. The method according to any one of the preceding claims, wherein the further nucleic acid of interest is chosen from a nucleic acid encoding a chimeric antigen receptor (CAR), Mcl-1, Bcl-xL, and Helios.

8. The method according to any one of the preceding claims, wherein the pluripotent stem cells are human induced pluripotent stem cells or human embryonic stem cells.

9. The method according to any one of the preceding claims, comprising an additional step of isolating a cell population chosen from regulatory T cells (Treg), effector T lymphocytes (Teff), and CD4-CD8-CD3+TCRab+ T lymphocytes.

10. The method according to any one of the preceding claims, wherein the population of T cells obtained by the method comprises regulatory T cells (Treg), effector T cells (Teff) and CD4-CD8-CD3+TCRab+ T cells.

11. The method according to claim 10, wherein the population of T cells obtained by the method comprises CD8+CD3+TCRab+Foxp3+ and CD4+CD3+TCRab+Foxp3+ Treg cells, CD8+ and CD4+ effector T cells, and CD4-CD8-CD3+TCRab+ T cells.
